# EUROPEAN PATENT SPECIFICATION

(11) **EP 0 863 155 B1**
(45) Date of publication and mention of the grant of the patent: **23.01.2002**
(21) Application number: 98102522.4
(22) Date of filing: 09.04.1993
(51) Int. Cl.: C07K 14/47, A61K 38/17

(54) **Suppression of T-cell proliferation using peptide fragments of myelin basic protein**
Unterdrückung der Proliferation von T-Zellen mittels Peptidfragmenten des basischen Proteins aus Myelin
Suppression de la prolifération des cellules T grâce à des fragments peptidiques de la protéine basique de la myéline

(30) Priority: 09.04.1992 US 865318
(43) Date of publication of application: 09.09.1998
(62) Divisional of application: 93912176.0
(73) Proprietor: AUTOIMMUNE, INC., Pasadena, CA 91103 (US)
(72) Inventor: Weiner, Howard M., Brookline, Massachusetts (US); Miller, Ariel, Ahuza, 34 750 Haifa (IL); Hafler, David Allen, West Newton, Massachusetts 02165 (US); Al-Sabbagh, Ahmad, Norwood, Massachusetts 02062 (US)
(74) Representative: Grünecker, Kinkeldey, Stockmair & Schwanhäusser Anwaltssozietät

(56) References cited:
- WO-A-95/08572
- WO-A-96/16085
- N KARIN ET AL.: "Reversal of experimental autoimmune encephalomyelitis by a soluble peptide variant of a myelin basic protein epitope: T cell receptor antagonism and reduction of interferon gamma and tumor necrosis factor alpha production" J. EXP. MED., vol. 180, December 1994, NEW YORK, NY, USA, pages 2227-2237, XP000590823
- R MARTIN ET AL.: "A myelin basic protein is recognized by cytotoxic T cells in the context of four HLA-DR types associated " J. EXP. MED. , vol. 173, January 1991, NEW YORK, NY, USA, pages 19-24, XP000578296
- R MARTIN ET AL. : "Diversity in fine specificity and T cell receptor usage of the human CD4+ cytotoxic T cell response specific for the immunodominant myelin basic protein peptide 87-106" JOURNAL OF IMMUNOLOGY, vol. 148, no. 5, 1 March 1992, BALTIMORE US, pages 1359-1366, XP002060072

## Description

### FIELD OF THE INVENTION

The present invention relates to peptides consisting of all or a portion of fragment 84-102 of hMBP, pharmaceutical compositions comprising said peptides and uses of said peptides.

### BACKGROUND OF THE INVENTION

Multiple Sclerosis (MS) is a chronic inflammatory disease of the white matter of the human central nervous system and is believed to be of autoimmune etiology. Regardless of its eciology, MS is accompanied by autoimmune attack of nerve tissue. For example, the disease is characterized by prominent T-cell and macrophage infiltrates into nervous tissue (i.e., the brain, spinal cord, peripheral nerves or associated cell types), demyelination and neurological dysfunction. Myelin basic protein (MBP) has been extensively studied by the present inventors, their co-workers and others as an autoantigen in the disease because of its role as an inducing agent in the major animal model of MS, experimental allergic encephalomyelitis (EAE), as well as its role in the human disease post-viral encephalomyelitis. In addition, the present inventors and their co-workers have studied MBP as a "bystander antigen" (Serial No. 843,752, supra).

A major hypothesis regarding the pathogenesis of MS is that T-cells reactive with myelin basic protein in the white matter of the CNS initiate the inflammatory process. Another hypothesis is that T-cells reactive with proteolipid protein (PLP) initiate the inflammatory process. The demonstration that activated T-cells specific for myelin basic protein (MBP) can be isolated from MS patients (Allegretta, M., et al., Science: 247: 778, 1990) further implicates MBP-reactive T-cells in the pathogenesis of the disease. The work of the present inventors also shows that MBP-reactive T-cells are involved in the pathology of the disease, subsequent to initiation of the inflammatory process. (As will be described in more detail below, the present inventors demonstrated that healthy individuals also often have MBP-specific T-cells, but unlike those of MS patients, MBP-specific T-cells from healthy individuals are not activated.)

The current treatments for MS are solely palliative and involve administration of drugs which act in a non-specific fashion to suppress the immune response in the subject. Examples of such drugs are cyclophosphamide, Imuran (azathioprine) and Cyclosporin A. Steroid compounds such as prednisone and methyl-prednisolone are also employed in many instances. These drugs have limited efficacy against MS. Use of such drugs is limited by their toxicity and by the fact that they induce "global" immunosuppression upon prolonged use, i.e., they also down-regulate the normal protective immune response to pathogenic microorganisms thereby increasing the risk of infection. In addition, patients that are globally immunosuppressed for prolonged periods of time run an increased risk of developing certain malignancies.

More details on the immunological processes occurring are known for experimental allergic encephalomyelitis (EAE), the primary animal model for MS. EAE can readily be induced in small mammals by immunization with myelin basic protein (MBP) in an appropriate adjuvant or by adoptive transfer through the injection of CD4⁺, MBP-reactive T-cells (Alvord Jr, E.C., et al. eds. in Experimental Allergic Encephalomyelitis: A Useful Model for Multiple Sclerosis, A. R. Liss, N.Y., 1984; Makhtarian, D.E., et al. Nature 305:356, 1984; Ben-Nun, A. et al. J. Immunol. 129:303, 1982). The T-cells that induce EAE in both mice and rats, termed encephalitogenic cells, specifically recognize peptides corresponding to the immunodominant regions of MBP. The presentation of these regions to the T-cells occurs on the surface of antigen-presenting cells (APCs) in association with unique Major Histocompatibility Complex (MHC) class II molecules. It should be emphasized that immunodominant regions of MBP, that is the portion of the protein most often recognized by MBP-reactive T-cells of the CD4⁺ type, differs depending on the species of the host mammal and may also differ depending on the species of MBP, despite the fact that the amino acid sequence of MBP exhibits very high interspecies homology. For example, as the present inventors and their co-workers have discovered, an immunodominant epitope of human MBP in humans is contained within the subsequence of the human MBP molecule comprising amino acids 84-102.

This is evidenced by the specificity of human T-cells isolated from individuals afflicted with MS (related patent application Serial No. 502,559 (WO-A-9 115 525) and Example 1 below). The immunodominant region of mouse MBP is amino acids 1-9 when administered to mice (Zamvil et al., Nature 324:258, 1986) and that of rat MBP is amino acids 68-88 when administered to rats (Burns et al., J. Exp. Med. 169:27, 1989). On the other hand, the immunodominant region of guinea-pig MBP in rats is located within residues 75-84 (Hashim, G. Myelin: Chemistry and Biology, Alan R. Liss, N.Y. 1980).

Based on the work done in the EAE system, alternative therapies are being developed for the treatment of autoimmune diseases in general and MS in particular. U.S. Patent Application Serial No. 65,794 (WO-A-8 810 307) filed June 24, 1987 (now abandoned) and co-pending International Patent Application PCT/US88/02139 (WO-A-9 108 760), filed June 24, 1988, now in national stage as United States Application Serial No. 07/460,852 and a continuation-in-part of this application, United States Application Serial No. 07/596,936 (WO-A-9 206 708), disclose that oral or enteral administration of whole myelin basic protein as well as disease-inducing and non-inducing fragments and analogs thereof is effective in suppressing acute monophasic EAE and are useful in suppressing MS symptoms when similarly administered.

The following co-pending commonly assigned patent applications are also of interest: U.S Patent Application Serial No. 454,806 filed December 20, 1989 (WO-A-9 108 760) discloses the aerosol administration of autoantigens, disease-suppressive fragments of said autoantigens and analogs thereof as an effective treatment for treating T-cell mediated autoimmune diseases such as MS.

United States Application Serial No. 07/487,732, filed March 20, 1990 (WO-A-9 112 816) entitled "Enhancement of the Down Regulation of Autoimmune Diseases by Oral Administration of Autoantigens" discloses synergists (enhancers) for use with oral administration of autoantigens, disease-suppressive fragments and analogs thereof as effective treatments for T-cell mediated autoimmune diseases.

United States Application Serial No. 07/843,752 (WO-A-9 112 816) discloses methods and compositions for treating autoimmune diseases orally or by inhalation by administering bystander antigens. Bystander antigens are tissue-specific antigens that are present at the locus of autoimmune attack and that have the ability upon their being orally administered to generate T-suppressor cells which in turn suppress immune attack at the afflicted tissue. Bystander antigens are not necessarily autoantigens and are not necessarily themselves the target of immune attack. (In fact, there is evidence that the immunosuppressive epitope(s) of an autoantigen are different from the immunodominant epitope(s) thereof, although immunodominant epitopes (which do elicit suppression upon oral administration) may act as bystander antigens in suppressing immune attack directed against other portions of the same antigen or portions of other antigens in the afflicted tissue.) However, bystander antigens must (a) be specific to the afflicted tissue and (b) possess the ability to elicit T-suppressor cells upon oral administration.

T-cell receptors are composed of two distinct chains of protein material. Certain T-cell receptors (TCRs), composed of V-beta (VB) chains and V-alpha (VA) chains, are known to recognize MBP. In SJL/PL mice, encephalitogenic (i.e., disease-inducing when administered to mice) T-cells having these receptors recognize an N-terminal mouse MBP peptide (residues 1-9) presented by an MHC molecule (Zamvil, S.S. et al., Nature 324: 258, 1986) encoded by the mouse gene H-2. The majority of T-cell receptors recognizing this peptide presented in connection with the MHC are encoded by the mouse TCR genes VB8.2 and VA2 or VA4. In Lewis rats, TCR gene segments that are homologous with the mouse VB8.2 and TCR VA2 genes have been found in encephalitogenic T-cells which recognize MBP residues 68-88 in the context of the Lewis rat MHC (Burns, F.R., et al., J. Exp. Med. 169: 27, 1989). Administration of a VB8.2-specific monoclonal antibody (i.e., an antibody recognizing the product VB8.2 expressed by the corresponding gene) to mice has been shown to be effective in treating murine EAE. Immunization with peptides specifically corresponding to the TCR VB8.2 amino acid sequence ameliorates EAE in the Lewis rat (Vanderbark, A.A., et al., Nature 341: 541-544, 1989; Howell, M.D. et al., Science: 246, 668; 1989).

Martin et al (J. Exp. Med. 173, 1991) discloses monospecific T-cell lines from MS patients which recognise a MBP peptide with the core sequence amino acid 89-99.

Martin et al. (J. Immunol. 148, 1992) discloses the presence of immunogenic epitopes in MBP using alanine substituted peptides.

However, the regions of an autoantigen (such as MBP) that behave as immunodominant regions are species specific. It has not heretofore been determined if common V-gene usage in TCR V-genes exists in humans among T-cells recognizing immunodominant regions of MBP nor have these immunodominant regions been positively identified in MS patients.

It has now been found that oral administration of multiple doses and small amounts of whole antigens containing encephalitogenic immunodominant epitopes elicits this type of active suppression. On the other hand, i.v. administration of entire autoantigen (or of one or more encephalitogenic immunodominant epitope-containing fragments thereof) also induces suppression but only of immune attack T-cells recognizing epitopes of the autoantigen. The latter type of suppression, which is believed to proceed via the mechanism of clonal anergy, is also observed upon oral administration of single doses and large amounts of antigens encompassing encephalitogenic epitopes, specifically immunodominant epitopes, especially when such antigens are accompanied by protease inhibitors.

For human MBP, a protein believed to be an autoantigen for MS, extensive testing by the present inventors has revealed fragments of the protein incorporating epitopes which are recognized by a large number of MBP-specific immune attack (CD4⁺) T-cells isolated from MS patients. Such fragments, comprising immunodominant epitopes, are likely candidates for administration to patients suffering from MS, with the goal of suppressing autoimmune response, and particularly suppressing the function of MBP-reactive T-cells that are responsible for autoimmune attack on nervous tissue. To that end, the present invention contemplates not only oral administration of such peptide fragments to mammals in need for such treatment but also parenteral administration of such fragments.

Therefore, it is an object of the present invention to provide immune-suppressive agents, specifically fragments of human MBP, and methods of using these fragments to suppress human T-cell functions.

Another object of the present invention is to provide compositions and pharmaceutical formulations comprising these fragments of human MBP useful for oral and/or parenteral administration to humans, and methods of use of such formulations.

Yet another object of this invention is to provide, as a reagent that could be used to determine the specificity of human T-cells, a peptide fragment of MBP incorporating an immunodominant epitope.

A further object is to provide compounds and compositions that anergize MBP-reactive T-cells or cause active suppression of such T-cells, the latter being evidenced for example by suppression of proliferation of MBP-reactive T-cells.

These and other objects of the present invention will be apparent to those of ordinary skill in the art in light of the present specification, drawings and claims.

### BRIEF DESCRIPTION OF THE DRAWINGS

Figure 1 is a bar graph showing the frequency of MBP reactive T-cells isolated from MS patients (left panel) and healthy controls (right panel) that react specifically with different human MBP peptide fragments.

Figures 2A and 2B graphically illustrate the suppression of EAE (induced by i.p. inoculation with MBP-specific encephalitogenic T-cells) by transfer of spleen cells from animals orally tolerized with MBP.

Figure 3 depicts the suppression of adoptively transferred EAE by co-transfer of CD4⁺-depleted or CD8⁺-depleted T-cells from MBP fed animals.

Figure 4 is a bar graph of DTH responses associated with the extent of protection (if any) against EAE induction by co-transfer along with CD4⁺ T-cells of various T-cell subsets from MBP fed animals.

Figure 5 is a bar graph of quantitative histologic analysis expressed in mean number of inflammatory foci isolated from the CNS (i.e., the parenchyma and the meninges) of mice injected with various T-cell subsets from MBP-fed mice.

Figure 6A depicts the suppression of actively induced EAE by intravenous (IV) administration of MBP; Figure 6B depicts the effect on adoptively transferred EAE of IV administration of MBP and the inability of spleen cells of IV-tolerized animals to confer suppression when they are co-transferred to naive animals along with an encephalitogenic MBP line.

Figure 7 is a bar graph showing the variation in suppression of EAE following oral (A) or IV (B) administration of different MBP peptides.

Figure 8 is a graphic representation of the ability of various peptides constructed based on the immunodominant epitope region of human MBP (human MBP amino acid residues Nos. 84-102) to stimulate proliferation of human MBP-reactive T-cell clones. Panel A: effect of omitting one or more N-terminal amino acids; Panel B: effect of omitting one or more C-terminal amino acids.

Figure 9 is a graph of the ability of a 15-mer (human MBP amino acid residue Nos. 85-99) to stimulate proliferation of four different human T-cell clones compared to the ability of MBP peptides 84-102 and 86-97 to stimulate such proliferation.

Figure 10 is a chart showing the TCR/MHC contacts for the 85-99 and 88-104 human MBP peptides and a proposed motif for this interaction.

Figure 11 is a chart showing the induction of proliferation of T-cell clones to the human 85-99 MBP peptides of the present invention as compared to the proliferation induced in these clones by the native MBP protein.

Figure 12 is an autoradiograph showing PCR amplification of cDNA's from 18 T-cell lines which were generated from five MS patients and which were reactive with MBP residues 84-102.

Figure 13 is an autoradiograph of a Southern blot analysis of TCR VB and JB gene usage for MBP-reactive T-cell lines generated from peripheral blood of an MS patient.

Figure 14 is a series of bar graphs showing the reactivity of T-cells isolated from MS patients and controls to different regions of the human MBP polypeptide in relationship to whether these patients have certain MHC antigens.

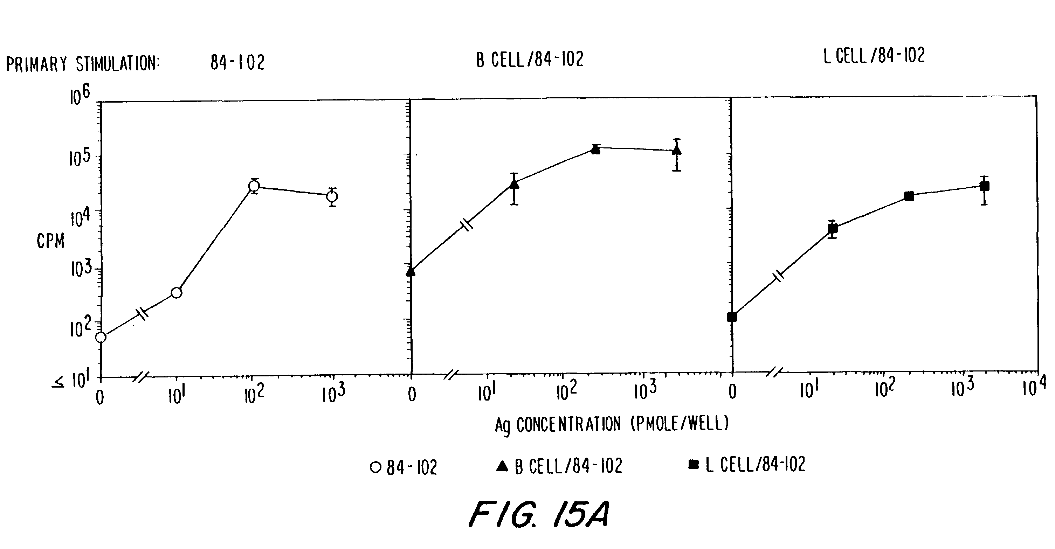

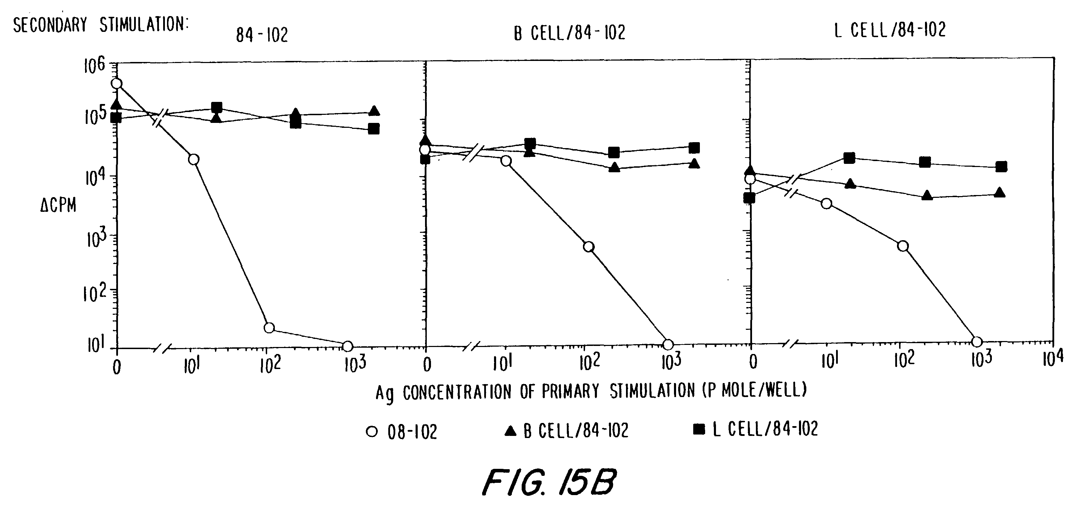

Figure 15. T-cells previously stimulated with free peptide antigen are unresponsive to antigenic stimulation. T-cell clone Ob.1A12.8 was stimulated either directly with MBP peptide 84-102 or with a DR2+ B cell line (9010) or L cell transfectant pulsed with 84-102 for 2 hours at 37°C and assayed for proliferation. Final concentration of peptide in wells of 1 stimulation is indicated. As seen in panel A, all three stimuli resulted in equivalent T-cell proliferation. Seven days later, T-cells were washed and re-assayed for response to 84-102 (5 µg/ml) or B cells or L cells pulsed with 84-102 (100 µg/ml). As shown in panel B, T-cells originally stimulated with high concentrations of 84-102 were unresponsive to any secondary antigenic stimulation.

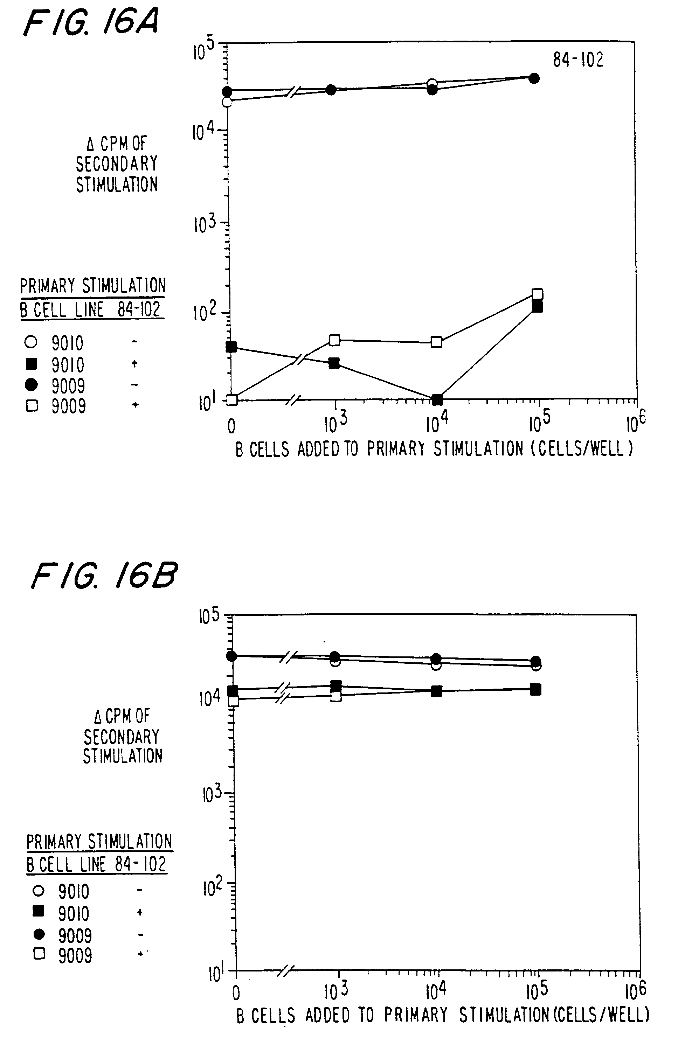

Figure 16. T-cell unresponsiveness can not be prevented by the addition of B cells. T-cell clone Ob.1A12.8 was cultured for seven days of primary stimulation either alone or in the presence of 84-102 with the addition of either MHC class II matched (9010) or mismatched (9009) irradiated transformed B cells at the numbers indicated, then washed and assayed for proliferation to either MBP 84-102 peptide (5 µg/ml) or rIL-2 (10³ u/ml). T-cells grown in the absence of peptide (circles) were fully able to proliferate to the secondary antigenic stimulation while those grown with peptide (squares) were antigen unresponsive regardless of the addition of B cells. Proliferation of IL-2 was equivalent in all cell populations.

Figure 17. Kinetics of T-cell anergy. T-cell clone Ob.1A12.8 was cultured for 0 to 168 hours in 5 µg/ml 84-102, washed, and assayed for proliferation to 84-102 or IL-2. Error bars represent SEM of CPM from triplicate cultures. Data from two consistent but separate experiments have been pooled to represent all time points.

Figure 18. Anergized T-cells continue to express CD3. T-cell clone Ob.1A12.8 was cultured in the presence or absence of 5 µg/ml 84-102 for 4 days, washed, and analyzed for both cell surface phenotype and proliferative response. Cells were stained with control MAb MsIg-FITC or OKT3 FITC (Coulter) for 30 min. at 4°C, washed twice, then fixed in 1% formalin and analyzed by flow cytometry. T-cells from the same primary culture were assayed for proliferation to B cells (9010) pulsed with or without 84-102, or rIL-2.

Figure 19. Activation of anergized T-cells by various stimuli. T-cell clone Ob.1A12.8 cultured in the presence or absence of 84-102 for seven days was washed and assayed for proliferation to logarithmic titrations of αCD3 + PMA, T11₂ + T11₃, PMA + iononmycin, and rIL-2. Final concentration or ascites dilution of each reagent is expressed on the x-axis. PMA concentrations are listed below concentrations of αCD3 or ionomycin.

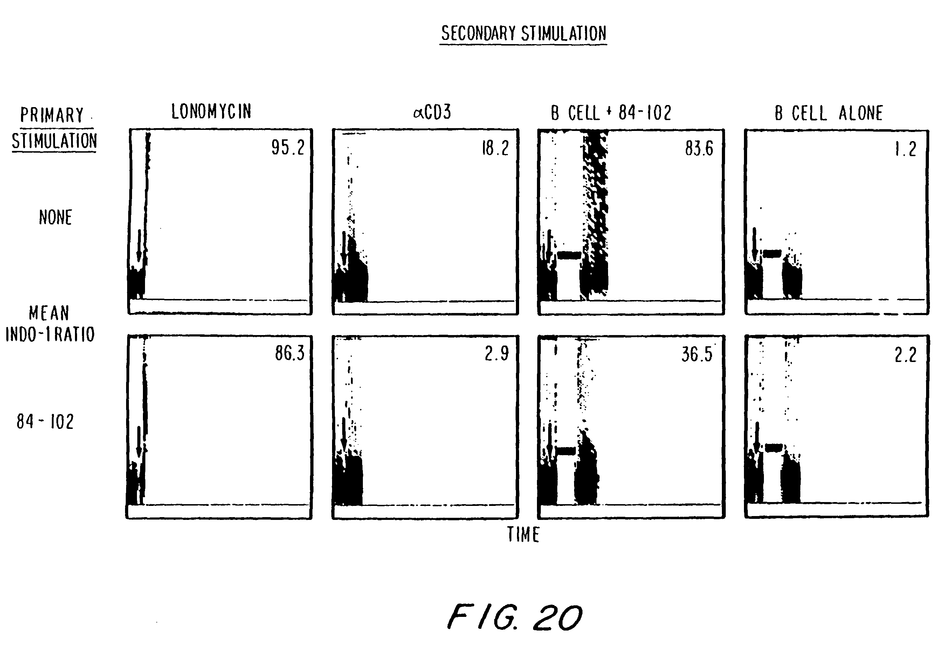

Figure 20. Anergized T-cells are inhibited in their ability to release [Ca⁺²]i to antigenic stimulation. Ob.1A12.8 was cultured ± 5 µg/ml 84-102 for 7 days, washed, and loaded with 2 µg/ml Indo-1 for analysis of [Ca⁺²]i. Secondary stimulation by ionomycin (100 µg/ml), αCD3 (1/30 asc dil.), or B cells (9010) pulsed + 100 µg/ml 84-102 was added to flow cytometer at point designated by arrows. For APC stimulation, cells were removed from flow cytometer and centrifuged for 1 min. (represented by solid bars). Percent response for each sample represents % of cells above baseline (post-stimulus minus pre-stimulus).

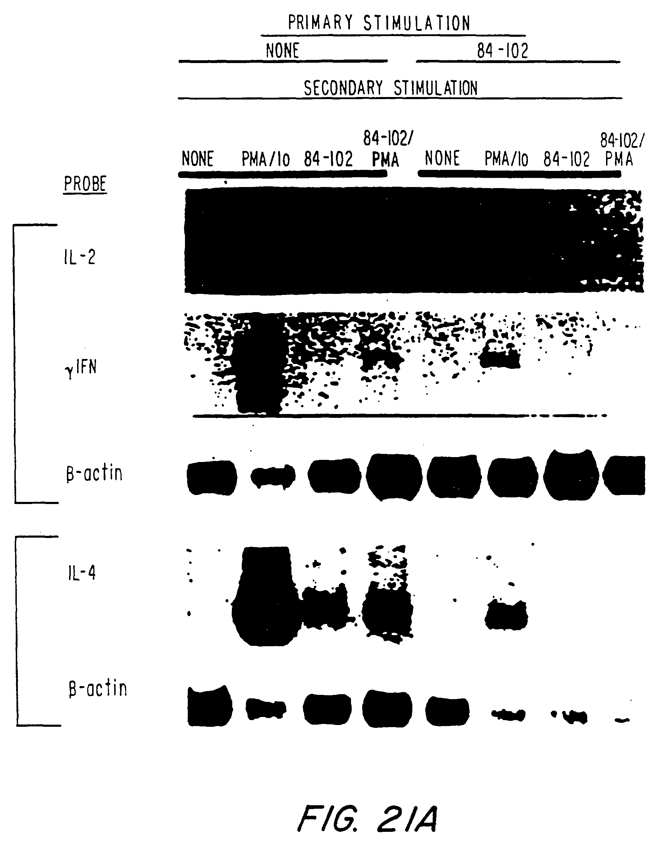

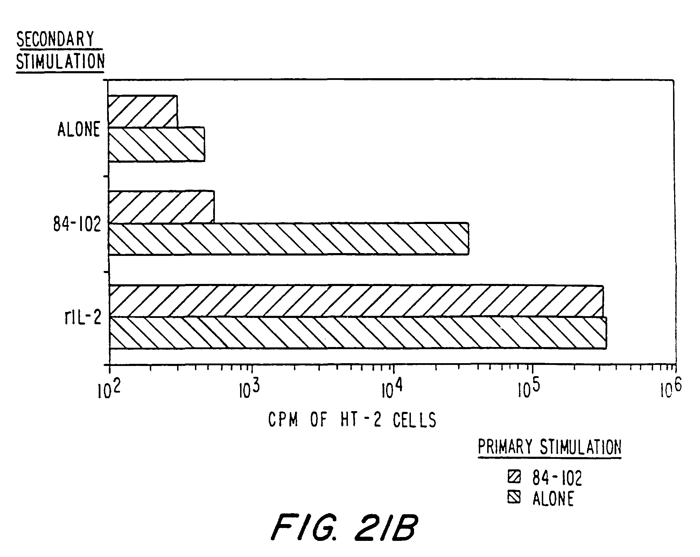

Figure 21. Cytokine production by anergized T-cells A. T-cell clone Ob.1A12.8 was cultured with or without 84-102 for 2 days, washed, stimulated with or without 10 ng/ml PMA +1 µg/ml ionomycin or 5 µg/ml 84-102 ± 10 ng/ml PMA for 4 hours at 37°C. Total cellular RNA was extracted by the RNAzol B method for northern analysis. Brackets signify 2 different northern blots of identical samples each hybridized with a β-actin control probe in addition to cytokine specific probes. Approximate sizes of mRNA bands are listed. B. Ob.1A12.8 was cultured for 2 days ± 84-102, washed, and stimulated with 84-102 or rIL-2 as a positive control. Supernatants were collected after 20 hours of secondary culture and diluted 1:3 in cultures of HT-2 cells. Proliferation of HT-2 cells represents the presence of IL-2 in supernatant of 84-102 stimulated nonanergized T-cells and was maximal with the rIL-2 positive control.

### SUMMARY OF THE INVENTION

The present invention provides peptides consisting of all or a portion of fragment 84-102 of hMBP containing an alanine residue at position 85, 86, 87, 88, 89, 90, 91, 92, 93, 94, 95, 96, 97, 98 or 99 and comprising at least fragment 85-99 of hBMP, said peptide having the property of stimulating in vitro proliferation of T-cells that are derived from MS patients and that recognise the entire fragment of hMBP.

Furthermore, the present invention provides a pharmaceutical composition comprising an orally effective amount of said peptides and a physiological acceptable carrier or diluent.

Furthermore, the present invention provides a pharmaceutical composition comprising a parenterally effective amount of said peptides and a physiological acceptable carrier or diluent.

In another embodiment the present invention provides the use of said peptides for preparing a medicament for altering the immune function of CD4T-cells reactive with myelin basic protein in a mammal afflicted with multiple sclerosis wherein the peptide is administered via the parental route and is administered in an amount effective to alter said immune function.

Furthermore, the present invention provides the use of said peptide for preparing a medicament for suppressing autoimmune response in a mammal, said autoimmune response being of the type observed in multiple sclerosis wherein the peptide is administered orally.

### DETAILED DESCRIPTION OF THE INVENTION

As used herein, "suppression" includes any measurable reproducible reduction in T-cell proliferation in response to factors that normally stimulate those cells. However, it should be emphasized that the present invention is concerned only with the suppression of proliferation of deleterious T-cells, that is proliferation of T-cells that promote autoimmune attack (CD4⁺ T-cells specific to a self-antigen, e.g. MBP). Indeed, an important aspect of the present invention is the ability to induce suppression in a restricted manner, where the suppression of deleterious T-cells specific to a self antigen is the result of choice of the fragment or fragments of MBP administered and/or the method of administration, as discussed below.

Suppression of the deleterious T-cell proliferation can also be measured indirectly, i.e. as seen through a reduction in symptoms of a disease which are directly or indirectly caused by immune attack T-cell proliferation, such as the damage to neural tissue observed in MS, or the decrease in the number or severity of attacks of MS suffered by MS patients. Damage to neural tissue can be assessed for example by magnetic resonance imaging (MRI) and measurement of the number and severity of lesions visible therein. Reduction in MS attack number or severity can be assessed for example by clinical evaluation of patients. Methods for both MRI and clinical evaluation are well-known in the art.

The term "autoantigen" is defined as any substance normally found within a mammal that, in an abnormal situation, is no longer recognized as "self" by the lymphocytes or antibodies of that mammal, and is attacked by the immunoregulatory system as though it were a foreign substance. In other words, an autoantigen is an antigen that is subject to autoimmune destruction. The mere presence of antibodies or even T-cells (of the CD4⁺ type) specific to a native antigen does not establish it as an autoantigen. MBP and PLP (proteolipid protein) are examples of autoantigens in MS.

"Immunodominant epitope" of an autoantigen (such as MBP) means an antigenic determinant recognized by a substantial number including but not limited to a majority (although not necessarily an absolute majority) of T-cells of a sensitive mammal to which autoantigen such T-cells will mount or help mount an immune response if the sensitive mammal is also an afflicted mammal. (It is evident from this discussion that a "sensitive" mammal need not be an afflicted mammal.)

"Immunodominant regions" or "immunodominant domains" of an autoantigen are defined herein as those regions of the amino acid sequence of such autoantigen containing an immunodominant epitope. The structures (and/or location within the MBP or other autoantigen molecule) of immunodominant epitopes (and regions) of MBP or other autoantigen vary depending on the host and are, therefore, host-specific. The present inventors have in fact adduced evidence that the reason the immunodominant epitopes are host-specific is that they must comprise a motif (believed to be contained within a peptide fragment of about 8 to about 15 amino acids in length) that binds to the major histocompatibility complex of the host. This motif varies among different species (the MHC likewise varies) and may also exhibit polymorphism among members of the same species.

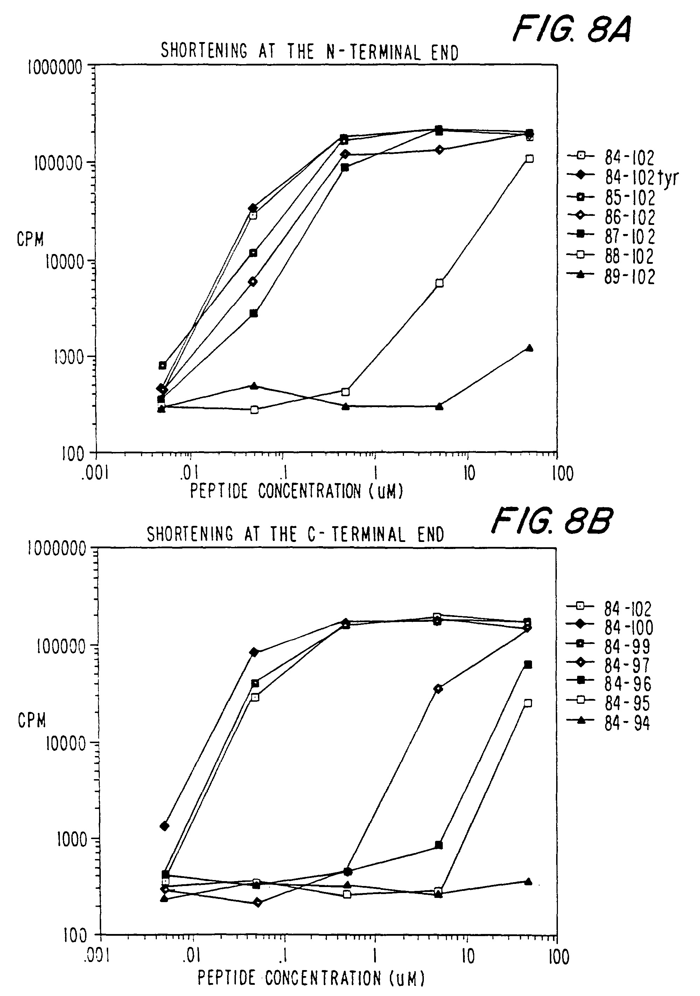

The term "analog" of fragments of MBP includes compounds that are so structurally related to the fragment of MBP that they possess the same biological activity as the MBP fragment. The biological activity referred to in this definition is the ability to suppress a T-cell mediated or T-cell dependent autoimmune response upon administration of the MBP fragment, or alternatively the ability to suppress proliferation of T-cells responsible for or contributing to autoimmune attack, or the ability to be recognized by T-cells recognizing an immunodominant epitope of MBP. An example of an analog of the fragment MBP 84-102 is the fragment MBP 84-102tyr, wherein amino acid 102 has been changed to tyrosine. As can be seen from Figure 8, this change has little or no effect on the ability of the MBP fragment to stimulate proliferation of the MBP-reactive T-cell line. Furthermore, amino acid substitutions are not expected to have any effect on solubility or pharmacokinetics of a fragment because of the relatively small size of the present fragments. It should be noted that an "analog" need not display the same activity to the same degree, e.g., an "analog" does not need to be as potent a suppressor as an actual fragment of the native antigen.

Other analogs of the relevant human MBP epitopes could be constructed based on ability of these analogs to bind the MHC and to be recognized by the relevant T-cell receptor (both of which can be tested in vitro).

As used herein, "T-cells" or "T-lymphocytes" are defined as immune system cells, derived from stem cells located within hematopoietic (i.e. blood forming) tissues. There are three broad categories of T-cells: Helper, Suppressor and Cytotoxic. T-cells express either the CD4 antigen (and are then termed CD4⁺ T-cells) or the CD8 antigen (in which case they are termed CD8⁺ T-cells) on their cell surface. The expression of CD4 or CD8 antigens by peripheral (circulating) T-cells correlates with the function and specificity of the T-cell. "Helper T-cells" which are CD4⁺ recognize antigens and Class II MHC molecules and perform helper or regulatory functions. "Cytotoxic" and "Suppressor" T-cells (which are CD8⁺) recognize antigens and Class I MHC molecules and perform cytotoxic and suppressor functions.

"Active suppression" is the suppression of immune function where the suppression is the result of the induction of additional immune cells, specifically, regulatory (suppressor) T-cells.

"Clonal anergy" is the suppression of immune function by induction in immune cells, specifically immune attack T-cells, of a state of unresponsiveness and more particularly unresponsiveness to presentation of the antigen to which these cells are normally specific and to which they would normally proliferate. La Salle, J. et al, J. Exp. Med., 176:177-186, July 1992. Anergized T-cells appear normal in all respects except they seem to be "turned off". They are not activated and - in the absence of added interleukin-2 (IL-2) - they do not proliferate on presentation of the antigen which they recognize. If IL-2 is added, the cells become de-anergized and begin to proliferate on presentation of antigen.

As used herein, "treatment" is meant to include both prophylactic treatment to prevent an autoimmune disease having the symptoms of MS (or the manifestation of clinical symptoms thereof) as well as the therapeutic treatment, i.e. the suppression or any measurable alleviation of one or more symptoms after the onset of a disease presenting the symptoms of MS.

"Receptor-derived peptides" are defined herein as peptides having the amino acid sequences of (or contained in) VB17 and/or VB12 of the T-cell receptor or analogs thereof as well as other agents (such as attenuated VB17- or VB12-containing T-cells), which when administered to a mammal suffering from a disease having the symptoms of MS will suppress one or more of such symptoms. (The minimum sequence length of the active peptides of the present invention is about 20 amino acids. There is no particular maximum as long as activity is preserved. For example, the entire TCR or even entire T-cells could be used. ;

"MHC" or "Major Histocompatibility Complex" is defined as a complex series of mammalian cell surface proteins present on the surface of activated T-cells, macrophages and other immune system cells. The MHC plays a central role in many aspects of immunity both in presenting histocompatibility (or transplantation) antigens and in regulating the immune response against conventional (foreign) antigens. There are two types of MHC protein molecules, class I and class II. The human MHC genes are located on human chromosome 6 and the mouse MHC genes are located in the H-2 genetic locus on mouse chromosome 17.

"Class II MHC molecules" are membrane glycoproteins that form part of the MHC. Class II MHC molecules are found mainly on cells of the immune system including B-cells, macrophages, brain astrocytes, epidermal Langerhan's cells, dendritic cells, thymic epithelium and helper T-cells. Class II MHC molecules are involved in regulating the immune response during tissue graft rejection, stimulation of antibody production, graft-versus-host reactions and in the recognition of "self" (or autologous) antigens, among other phenomena. In the specification below, MHC shall be used interchangeably with "Class II MHC". The MHC genes will be referred to as "MHC genes". T-cells initiate an immune response when they encounter antigen-presenting cells (APCs), such as mononuclear phagocytes (macrophages, monocytes), Langerhan's cells or follicular dendritic cells, which initially take up, process (digest) and present antigenic fragments of a protein on their cell surface (in connection with their MHC). CD4⁺ T-cells recognize antigen molecules exclusively when the protein is processed, and peptide fragments thereof are presented, by APCs that express Class II MHC molecules.

"T-cell receptor" or "TCR" is defined herein as the antigen recognition receptor present on the surface of T-cells TCR is, therefore, the receptor that binds a molecule which the immune system recognizes -- and presents -- as an antigen (whether the molecule is foreign or autologous, the latter being the case in an autoimmune disease). A majority of T-cells express a TCR composed of a disulfide-bonded heterodimer protein containing one alpha (A) and one beta (B) chain whereas a minority of T-cells express two different chains (gamma and delta). The TCR is composed of an A and a B chain, each of which comprises a variable and a constant region. (Tilinghast, J.P. et al., Science 233: 879, 1986; Concannon, P. et al., Proc. Natl. Acad Sci USA 83: 6589, 1986, Kimura, N. et al., J. Exp. Med. 164: 739, 1986; Toyonaga, B. et al., Proc Natl. Acad. Sci USA 82: 8624, 1985.) The variable region in turn comprises a "variable", a "diversity" and "joining" segment. The junction among the variable, diversity and joining segment is postulated to be the site of antigen recognition by T-cells. See also, Ho, H. Z., etal, Immunogenetics, 1982, 15:509-517; Olerup, O. et al, Tissue Antigens, 1987, 30:135-138; Opelz, G., et al, Tissue Antigens, 1977, 9:54-58; Danska, J.S., et al, J. Exp. Med., 1990, 172:27-33; Kempkis, B., et al, J. Immunol., 1991, 147:2467-2473; Sellins, K. S., et al, J.Immunol., 1992, 149:2323-2327.

T-cell recognition of an antigen reflects a tri-molecular interaction between the T-cell receptor (TCR), the MHC molecule of the APC and a peptide or peptides processed by the APC via a cleft or pocket in the three-dimensional structure of the Class II MHC molecule. (Bjorkman, P.J., et al., 1987, Nature, 329:506 and 329:512). The portion of the protein most often presented on the APC surface, and recognized by the T-cell is the immunodominant epitope.

In the animal model (EAE) T-cell receptors comprising a portion of the animal VB8.2 sequence have been used to treat the disease and shown to act by eliminating disease-inducing T-cells. In particular, in the animal model, peptides comprising the sequences Thr-Leu-Cys-Ala-Ser-Ser and Thr-Leu-Cys-Ala-Ser-Arg which may correspond to exposed (surface) portions of mouse and rat VB8.2 have been determined (in mouse and rat models) to combat the autoimmune disease model by eliminating Helper T-cells.

The present inventors have identified a region of human MBP which contains an immunodominant epitope of human MBP in a human host. The epitope is resident within a distinct portion of the human MBP amino acid sequence (residues Nos. 82-104. As shown in Example 1 below, the present inventors have identified human MBP amino acid residues Nos. 84-102 as one immunodominant domain of human MBP recognized by a majority of peripheral T-cells isolated from patients suffering from MS. Additional experimentation has determined that the immunodominant epitope within this domain is further localized within human MBP amino acids Nos. 85-99. These data are presented in Example 3. (It appears by inference from the data in Example 3, that the minimal human MBP fragment within which the foregoing human host immunodominant epitope may reside is fragment 87-98 for some T-cell clones. But all T-cell clones reactive with 84-102 recognize the fragment 85-99.)

Experiments involving the animal model of MS, EAE, have shown that protein fragments including corresponding immuno-dominant epitopes of guinea pig MBP and bovine MBP in rats, when administered orally to animals suffering from the disease, are effective in the suppression of the symptoms of the disease (related patent application Serial No. 07/596,936 and Example 2 below) although some noninducing fragments are more potent suppressors than inducing fragments. Further, it has been shown that this orally effected suppression is due to the induction of CD8⁺ suppressor T-cells (Lider et al., J. of Immunol. 142:748, 1989). Some experiments have been conducted by others in animals using encephalitogenic fragments of MBP: See, e.g., Swierkosz, J.E., 1977, J. Immunol. 119:1501-1506; Su, X-M et al., 1991, J. Neuroimmunol. 34:181-190 (i.v. use of MBP fragments - determined to be encephalitogenic in mice - coupled'to spleen cells to abate adoptively transferred EAE in mice); Avrilionis, K. et al., 1991, J. Neuroimmunol. 35:201-210 (i.v. use in guinea pigs of liposome-bound human MBP peptide fragment - shown to be encephalitogenic when administered to guinea pig - to suppress EAE induced with the same fragment and i.p. and s.c. use of the free peptide for the same purpose).

Thus, the peptides of the present invention can be advantageously used in the design of specific immunosuppressive preparations containing such peptides which are in turn useful for the suppression of deleterious T-cell proliferation. For example, peptides comprising sequences of the human MBP shown to induce anergy in human MBP-specific CD4⁺ T-cells or to induce T-suppressor cells specific for demyelination can be constructed and used for such purposes. See, e.g., Examples 1 and 2 below. Further, the results reported in Example 2 indicate that the method and protocol of administration of the tolerizing agents affect the mechanism which brings about the suppression of the autoimmune reaction. Thus, peptide fragments incorporating an immunodominant epitope of human MBP in humans are effective in inhibiting proliferation of MBP-reactive CD4⁺ human T-cells in vitro and are anticipated to be effective by the same mechanism when administered via i.v. route in humans. The same epitopic peptides are anticipated to be effective in inducing suppression of autoimmune attack of human neural tissue when administered to humans orally. The present inventors also have evidence that a whole MBP (which encompasses the foregoing two immunodominant epitope regions) can induce suppression via elicitation of suppressor T-cells (active suppression) when MBP is orally administered in small amounts and in multiple doses. The same antigen, also administered orally but in high amounts and a single dose will induce suppression via anergy. Finally, there is evidence that both mechanisms of suppression can be triggered by adjustment of the oral administration protocol from MBP between the two extremes identified above.

Without wishing to be bound by theory, it is believed that the oral or enteral administration of immunodominant fragments of MBP can cause suppressor T-cells to be elicited that in turn suppress the T-cells that contribute to the autoimmune attack of a neural tissue (i.e., the brain, spinal cord, peripheral nerves or associated cell types). Nerve tissue damage constituting the pathology seen in patients suffering from MS is believed to be the direct result of such an autoimmune attack. As this tolerizing mechanism involves the active induction of regulatory (suppressor) T-cells responsible for the suppression of the immunoreactivity of cells in the vicinity of tissue under immune attack, it is an example of active suppression.

The present inventors have accumulated a large body of experimental evidence that active suppression takes place by the elicitation of tolerizing-antigen specific T-suppressor cells which are targeted to the locus (locuses) of the body where the antigen to which these T-suppressors are specific can be found. This locus includes the tissue under autoimmune attack. Once they encounter this antigen, the T-suppressors secrete suppressive cytokines such as the non-specific immunosuppressive factor TGF-β, and interleukin-4 (IL-4) which suppress autoimmune responses including autoimmune attack. (See related patent application Serial No. 843,752.) (WO-A-9 316 724).

In contrast, intravenous administration (or subcutaneous, or intraperitoneal administration) of the MBP fragments incorporating immunodominant epitopes is believed to bring about immune suppression through another mechanism known as clonal anergy. Clonal anergy, or T-cell unresponsiveness, has been attributed to antigen presentation in the absence of the appropriate co-stimulatory factors. Jenkins, M.K. PNAS, 84:5409-5413, 1987. The exact identity of the factors involved is ill-defined, but soluble cytokines (e.g. B-7, EDCI, and an appropriate intracellular calcium concentration) have been implicated. More recent evidence, however, suggests that so-called "negative signals" rather than the absence of co-stimulatory factors are responsible for anergy. However, these signals have not yet been defined. See, LaSalle J.M. et al, J. Exp. Med., 176:177-186, June 1992. Rather than inducing the T-cell clones to proliferate, presentation of antigen by the APCs without the co-stimulatory factors (and/or in the presence of the negative signals) causes the T-cells to become unresponsive to subsequent antigen stimulation, while remaining responsive to IL-2, and are thus described as being anergized (Jenkins et al., Proc. Natl. Acad. Sci. 84:5409, 1987; Mueller et al. Ann. Rev. Immunol. 7:455, 1989; Schwartz et al., Science 248:1349, 1990). Thus, autoimmune response-promoting clones specific to an autoantigen such as MBP, will no longer proliferate in response to that antigen, reducing the immune attack reactions which cause the tissue damage responsible for the autoimmune disease symptoms, such the neural tissue damage observed in MS.

Suppression by clonal anergy can be differentiated (and has been so differentiated in the experiments below) from active suppression by adoptive transfer experiments which test the ability or inability of suppressor T-cells transferred from a tolerized animal to a nontolerized animal to bring about suppression of immune function in the latter animal. T-cell transfer brings about suppression if the suppression mechanism is active, i.e. if it involves elicitation of T-suppressor cells and does not occur if the suppression mechanism is passive, i.e. if clonal anergy is involved. The results reported in Example 2 illustrate instances in which each mechanism of immune suppression is involved and show that the suppression mechanism depends on one or more of the following: (i) on the substance being administered to induce tolerance (for example, immunodominant epitopic fragments of MBP induce active suppression via the oral route and energy via the parenteral route); (ii) on the route of administration of the tolerizing antigen (for example, only epitopes that are recognized by attack T-cells induce anergy via i.v. route); and (iii) on the amount and frequency of administration (for example, orally administered MBP induces active suppression when given in small amounts and multiple doses and passive suppression when given orally in large amounts and a single dose).

The data show that both encephalitogenic and nonencephalitogenic fragments of MBP can elicit active suppression when orally administered, with those non-encephalitogenic fragments which incorporate an immunosuppressive epitope working only via active suppression and only when administered via the oral route. The encephalitogenic fragments (incorporating an immunodominant epitope) may also elicit anergy via the oral route if administered in high amounts and single doses.

These results have important ramifications on the design of tolerizing agents and methods based on MBP as a tolerizer. Thus, depending on the type of immune suppression desired, particular methods of administration, as well as particular fragments, may be used. For example, it may be desirable to use one or more disease-propagating epitopic peptides via the i.v. or s.c. or i.p. route and (concurrently) one or more immunosuppressive epitopic peptides via the oral route.

It is also anticipated that tolerizing methods involving combinations of administration routes, and/or protocols and/or autoantigen fragments may prove to be most effective. As will be understood by those skilled in the art, the effectiveness of a fragment or combination of fragments (or a combination of a fragment and whole antigen) and the effectiveness of a method (or combination of methods) of administration of a MBP fragment is a function of the age, sex, weight, physical condition, and disease stage of the subject to be treated, and the concurrent use or absence of other fragments or treatments. Consequently, the fragment(s) used and the method of administration must be determined in substantial part based on these factors, and may need to be determined experimentally on a case by case basis. Such determination, however, requires no more than routine experimentation, given the examples and guidelines contained herein.

Peptides based on the sequences of human MBP for use in the present invention, as identified in Example 1, can be synthesized using well-known solid phase methods (Merrifield, R.B. Fed. Proc. Soc. Ex. Biol., 21: 412, 1962 and J. Am. Chem. Soc 85:2149, 1963; R. Mitchell A.R. et al, J. Am. Chem. Soc. 98:7357, 1976; Tam, J. et al., J. Am. Chem. Soc. 105:6442, 1983), preferably using a commercially available peptide synthesis apparatus (such as that made by Applied Biosystems, Foster City, CA) and following the manufacturers' instructions. Alternatively, such peptides can be synthesized by recombinant DNA techniques as is now well-known in the art (Maniatis et al., Molecular Cloning: A Laboratory Manual, Cold Spring Harbor Laboratories, NY, 1982, see pp. 51-54 and pp. 412-30). For example, these peptides can be obtained as DNA expression products after incorporation of DNA sequences encoding the desired fragment of MBP isolated from the desired species into expression vectors and introduction of such vectors into suitable eukaryotic or prokaryotic hosts that will express the desired peptides individually or as part of fusion peptides or proteins, from which they can be later isolated using well-known techniques.

Peptide analogs can be designed using the known amino acid sequences encoded by the human MBP gene as disclosed below, using the synthetic or recombinant techniques described above and the methods of, e.g., Eyler, E.B., in Advances in Experimental Medicine and Biology 21: 259-281, 1978. For example, a peptide having a sequence based upon but differing from the exact amino acid sequence of the desired fragment of MBP can be chemically synthesized using the above-described techniques. Such a peptide can be tested for its effect on MBP-reactive CD4⁺ T-cells using e.g. the procedure described in Example 1 for identifying a more precise location within amino acids 84-102 for this particular immunodominant epitope of human MBP or the procedure described in Example 4 for testing binding to the T-cell receptor and to the MHC. An MBP-based peptide can be tested in vitro for effectiveness orally in humans by exposing collected, isolated peripheral MBP-specific suppressor T-cells from individuals to the peptide to determine whether they proliferate. Additionally, or alternatively, these isolated suppressor T-cells can be tested to determine whether they release suppressive cytokines such as TGF-β and/or IL-4 upon exposure to an MBP peptide. (See, e.g. the use of the transwell system in co-pending U.S. application Ser. No. 843,752 (WO-A-9 316 724) corresponding to PCT US92/01705 except that the use of spleen cells as APC's is not necessary; the MBP peptide can be used to induce the cells to release TGF-β.) MBP specific T-suppressor cells can be isolated by exposure to MBP and assessment of proliferation followed by binding studies using anti-CD8⁺ antibody.

The present invention also provides pharmaceutical formulations and dosage forms for oral or parenteral use in the suppression of autoimmune attack T-cell function in humans, particularly those subjects suffering from MS. In general such dosage forms contain one or more peptides according to the invention which are fragments of human MBP and analogs thereof, in an amount effective to suppress proliferation of immune attack cells. Suppression of function which results in an in vitro suppression of immune attack cells, such as MBP-specific CD4⁺ T-cells and/or attenuation of one or more symptoms of MS in a patient that has been treated pursuant to the method of the present invention is considered to be within the scope of the invention. See definitions section above for what constitutes suppression and symptoms attenuation.

The T-cell suppressive peptides of the present invention may also encompass additional non MBP-derived amino acid sequences leading or following the MBP-based sequences as long as these additional sequences do not defeat the suppressive function of such peptides. Testing of such constructs for immunosuppressive activity can be easily done using, for example, one or more of the assay methods described herein.

The pharmaceutical formulations of the present invention may include, as optional ingredients, pharmaceutically acceptable vehicles, carriers, diluents, solubilizing or emulsifying agents, and salts of the type that are well-known in the art. Nonlimiting examples of such substances include 0.5N saline in distilled water for parenteral use and lactose for oral use.

The fragments of human MBP can be administered orally or by-inhalation in conjunction with synergists which may enhance the effectiveness of the immune suppression. Non-limiting examples of synergists for use in the present invention include bacterial lipopolysaccharides from a wide variety of gram negative bacteria such as various subtypes of E. coli and Salmonella (LPS, Sigma Chemical Co., St. Louis, MO; Difco, Detroit, MI; BIOMOL Res. Labs., Plymouth, PA), Lipid A (Sigma Chemical Co., St. Louis, MO; ICN Biochemicals, Cleveland, OH; Polysciences, Inc., Warrington, PA) and immunoregulatory lipoproteins, such as peptides covalently linked to tripalmitoyl-S-glycarylcysteinyl-seryl-serine (P₃ C55) which can be obtained as disclosed in Deres, K. et al. (Nature, 342:561-564, 1989) or "Braun's" lipoprotein from E. coli which can be obtained as disclosed in Braun, V., Biochim. Biophys. Acta 435:335-337, 1976. LPS is preferred and Lipid A particularly preferred. Lipid A is particularly preferred for use in the present invention because it is less toxic than the entire LPS molecule. LPS for use in the present invention can be extracted from gram-negative bacteria and purified using the method of Galanes et al. (Eur. J. Biochem. 9:245, 1969) and Skelly, R.R., et al. (Infect. Immun. 23:287, 1979).

The effective amount of a synergist, e.g. LPS or Lipid A, to be administered in conjunction with the MBP fragment broadly ranges between about 0.15 and 15 mg per kg body weight of said mammal per day and preferably between about 0.3 and 12 mg per kg body weight of said mammal per day.

The route of administration of the suppressive agents of the present invention is in an oral or parenteral form or combinations thereof. Oral administration includes oral, enteral or intragastric administration with oral being preferred Parenteral administration includes intraperitoneal, subcutaneous, intradermal and most preferably intravenous administration routes.

In general, the MBP-based peptide or analog is administered orally to a human patient in an amount ranging between about 10 µg and about 20 mg per administration. preferably between about 100µg and 250µg. The amount is pulse-administered in a single dosage form or multiple dosage forms. For whole MBP to elicit active suppression a dosage of 1 mg five times daily is an example of an effective amount. For anergy, 10-20 mg of MBP parenterally are examples of an effective amount. Monitoring of the patient is desirable in order to optimize the dosage and frequency of administration. The exact amount and frequency of administration to a patient may vary depending on the stage, frequency of manifestation and severity of the patient's disease and the physical condition of the patient, as is well-appreciated in the art. Such optimization is preferably effected on a case-by-case basis. Optimization of the dosage necessary for immune suppression does not represent undue experimentation, given the guidelines disclosed herein.

In an alternative preferred embodiment of the present invention pharmaceutical oral formulations or dosage forms according to the present invention can also be administered by inhalation, preferably in aerosol form. The inhalation mode of administration is preferably not through the nasal passages but through the bronchial and pulmonary mucosa. The MBP fragment and related compounds of the present invention can be administered to humans as dry powder particles or as an atomized aqueous solution suspended in a carrier gas (e.g. air or N₂). Preferred aerosol pharmaceutical formulations may comprise for example, a physiologically acceptable buffered saline solution.

The use according to the present invention may involve by inhalation administration of pharmaceutical formulations in the form of an aerosol spray using for example, a nebulizer such as those described in U.S. Patent Nos. 4,624,251 issued November 25, 1986; 3,703,173 issued November 21, 1972; 3,561,444 issued February 9, 1971 and 4,635,627 issued January 13, 1971. The aerosol material is inhaled by the subject to be treated.

Other systems of aerosol delivery, such as the pressurized metered dose inhaler (MDI) and the dry powder inhaler as disclosed in Newman, S.P. in Aerosols and the Lung, Clarke, S.W. and Davia, D. eds. pp. 197-224, Butterworths, London, England, 1984, can be used when practicing the present invention.

Aerosol delivery systems of the type disclosed herein are available from numerous commercial sources including Fisons Corporation (Bedford, MA), Schering Corp. (Kenilworth, NJ) and American Pharmoseal Co. (Valencia, CA).

It is expected that lower amounts of the fragment of MBP of the present invention will be required using aerosol administration for treatment as this effect has been found when treating EAE with whole MBP and adjuvant arthritis with collagen as disclosed in co-pending U.S. Patent Application Serial No. 454,486 filed December 20, 1989(US-A-4 980 014). Further, it appears that the immune suppression induced by inhalation of the fragment of MBP occurs through the active suppression mechanism, similar to oral administration. Weiner, H. L. et al FASEB 4(7):2102, 1990. The amounts of the fragment of MBP of the present invention which may be administered in an aerosol dosage form would be between about 0.015 mg and about 1.5 mg per kg body weight of a mammal per day and may optionally include a synergist in amounts ranging between about 0.05 and about 15 mg per kg body weight of said mammal per day and may be administered in single dosage form or multiple dosage forms. The exact amount to be administered will vary depending on the state and severity of a patient's disease and the physical condition of the patient.

Dosage forms for parenteral administration will generally contain from about 1 to about 200 mg of a peptide according to the present invention per dose per person and preferably about 10 mg to about 100 mg. The foregoing description of inert optional ingredients and fine-tuning of amounts and administration scheduling given above with respect to oral formulations pertain to parenteral formulations only.

It will be appreciated that the unit content of active ingredient or ingredients contained in an individual oral or parenteral dose of each dosage form need not in itself constitute an effective amount for treating MS since the necessary effective amount can be reached by administration of a plurality of dosage units.

The techniques described below in Examples 1-3 can be used to monitor the effectiveness of the methods of the present invention and optimize the amount and frequency of administration of the disease suppressive agents of the present invention, as well as the fragments and method of administration used.

T-cells can be isolated from a patient's peripheral blood or cerebrospinal fluid, amplified and cloned as described in Examples 1-3 (before and/or after treatment according to the present invention). Antibodies (either polyclonal or monoclonal) can be obtained directed against the MBP peptides of the present invention (using conventional techniques well known and used in the art) to assay for the presence of MBP-reactive T-cells in a patient's peripheral blood and more specifically for the presence of activated MBP-reactive CD4⁺ T-cells before and/or after treatment according to the present invention. The peptides of the present invention are also useful in identifying individuals with T-cells reactive with MBP, using the method of Example 1.

The present invention is described further below in specific working examples which are intended to illustrate the present invention without limiting its scope.

### EXAMPLE 1: IDENTIFICATION OF THE MAJOR IMMUNODOMINANT REGION OF HUMAN MBP

MBP was extracted from human brain tissue and purified on a CM-52 column (Supplier: Wattman Biosystems Ltd Maidstone, Kent, U.K.) using the highest molecular weight peak (18kD) as described (Chou, F.C.-H. et al., J. Biol. Chem. 251:2671, 1976). MBP peptides were synthesized using a solid phase method or were obtained from a commercial laboratory (Biosearch Lab Inc., San Raphael, CA) and were purified by high pressure liquid chromatography as follows: Each peptide containing preparation was made up in 0.1% trifluoroacetic acid (TFA) at 1mg/ml. It was then processed in an HPLC column (Rainin Reverse Phase C4 or C18) using a 0-70% acetonitrile gradient containing 0.1% TFA. Peaks were detected at 214 nm. The MBP peptide fragments used are set forth below in Table 1. However, the sequence of human MBP is published. Therefore, only the numbers designating the amino acid residues contained in each peptide are necessary.

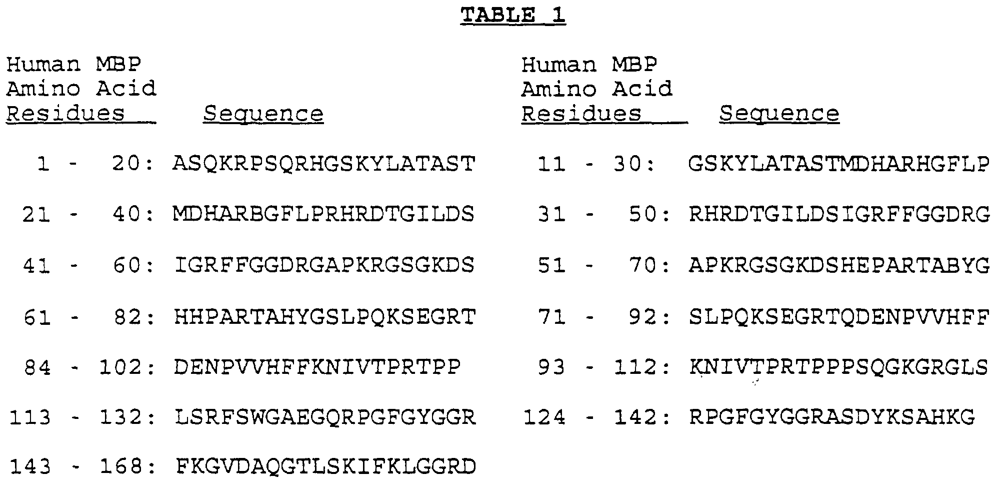

A rapid T-cell cloning technique was used to examine whether there were immunodominant epitopes on human MBP reactive with Class II MHC phenotypes and the frequency of such reactivity. A total of 15,824 short term T-cell lines were generated from 51 human subjects by culturing peripheral blood mononuclear cells (PMN) with purified MBP (100 µg) followed 3 days later, and then every 3-4 days, by the addition of interleukin-2 (IL-2; from ABI, Columbia, MD) and 2 units recombinant interleukin-4 (IL-4; Genzyme, Boston, MA). On Day 13 of culture, an aliquot from each line was tested for reactivity to MBP using the following proliferation assay: T-cell clones (1 x 10⁵ cells/well) were plated in triplicate and cocultured with appropriate stimuli (i.e. 100 µg whole MBP or 5% IL-2) for 72 hours at 37° C, 90% humidity, 5% CO₂, in 96 well flat bottomed microtiter plates (Costar). The cells were pulsed with 2 µCi [³H]TdR (2 Ci/mmole, New England Nuclear, Boston, MA) for the last 18 hours of culture. APCs were prepared by pulsing human Epstein-Barr virus transformed human B-cells (the virus being commercially available from ATCC) or L-cells which are mouse cells transfected with human DR₂ (L-cells are commercially available from ATCC under accession No. ATCC-CCL1 and can be transfected according to the method of Wilkinson, D. et al, J. Exp. Med., 1988, 167:1442-1458 using DNA encoding DR2 as per Wu, S. et al J. Immunol., 1987, 138:2953). B-cells or L-cells were used at 1 x 10⁶ cells/well in complete media either in the presence or absence of 40 pM MBP or phospholipid protein (PLP) for 2 hours at 37°C, washing twice with 4°C Hanks (Whittaker), followed by irradiation with 5000 rad. at 4°C. To stimulate T-cells without accessory APCs, 2 µM of MBP, PLP, or the appropriate fragment was added directly to the cells for the duration of the culture: 48 hours, followed by pulsing thymogen and then harvesting. Ten thousand APC's were used for 100,000 T-cell clones.

T-cell lines shown to be reactive to MBP or PLP were then tested using the same technique for reactivity to overlapping oligopeptide 20-mers encompassing the human MBP sequence as shown in Table 1 above. MBP and PLP reactivity frequency analysis was performed on patients with definite, relapsing-remitting MS (as diagnosed by Magnetic Resonance Imaging (MRI) and clinical examination), as well as on subjects with other neurologic diseases and on normal subjects (all age- and sex-matched to the MS patients). The results are shown in Table 2 below.

**TABLE 2**

| | AGE | SEX (%) (M/F) | #Ag REACTIVE LINES TOTAL # LINES | | MEAN FREQUENCY OF Ag REACTIVE LINES | |
|---|---|---|---|---|---|---|
| | | | MBP | PLP | MBP | PLP |
| MS* (n=23) | 34.2±1.4 | 35/65 | 554/7746 | 20/432 | 7.18±2.38 | 3.34±1.56 |
| OTHER N* DISEASE (n=10) | 38.7±3.2 | 43/57 | 118/2880 | 3/384 | 4.10±1.04 | 0.90±0.62 |
| NORMAL (n=6) | 30.3±1.5 | 50/50 | 73/1742 | ND | 4.70±1.58 | ND |

| | | | | | | |
|---|---|---|---|---|---|---|
| * MS = Multiple Sclerosis ** N = Neurological | | | | | | |

Patients with MS were caucasian and had well-characterized relapsing remitting disease with at least two exacerbations within the previous 24 months and positive lesions as seen using MRI at the time of blood drawing. Subjects with other central nervous system diseases had the following diagnoses: 1-3 weeks after either cerebrovascular accident (n=4), brain trauma with CNS hemorrhage (n=4), or metastatic brain tumor (n=2). The total number of T-cell lines reactive with MBP or PLP and the total number of T-cell lines generated are shown in Table 2 ("Ag" means "antigen"). In addition, the frequency of MBP and PLP-reactive lines was calculated separately for each subject by dividing the number of MBP-reactive lines by the total number of lines generated and the mean values +/- SEM are given. The same conclusions can be drawn regarding reactivity to PLP although to a lesser extent than reactivity to MBP or its fragments.

While the frequency of lines reactive to all fragments of MBP was slightly higher in subjects with MS as compared to the other subjects, this increase was not statistically significant. However, as discussed below, a significantly greater number of the MBP reactive cells lines from MS patients were reactive with the fragments including amino acids 84-102 thus identifying these peptides and the corresponding fragments of MBP as containing immunodominant epitopes of MBP. active in the development of MS.

Of a total of 302 cell lines from patients with MS that could be expanded and confirmed to react with MBP on repeated analysis, 140 (46.4%) reacted with MBP residues 84-102; and approximately 70-80% reacted with either MBP residues 84-102 or 143-168. In the control groups, 11 of a total of 100 MBP reactive T-cell lines (11.0%) recognized the 84-102 peptide and about 34% recognized either the 84-102 or the 143-168 peptide. The actual frequency of T-cells derived from the peripheral blood that reacted with each MBP peptide for each individual subject was calculated. The mean values for patients with MS and the control subjects are shown in the rightmost column of Table 2. The corresponding immunodominant peptide(s) of PLP can be identified by the same methods as described herein for the MBP peptides.

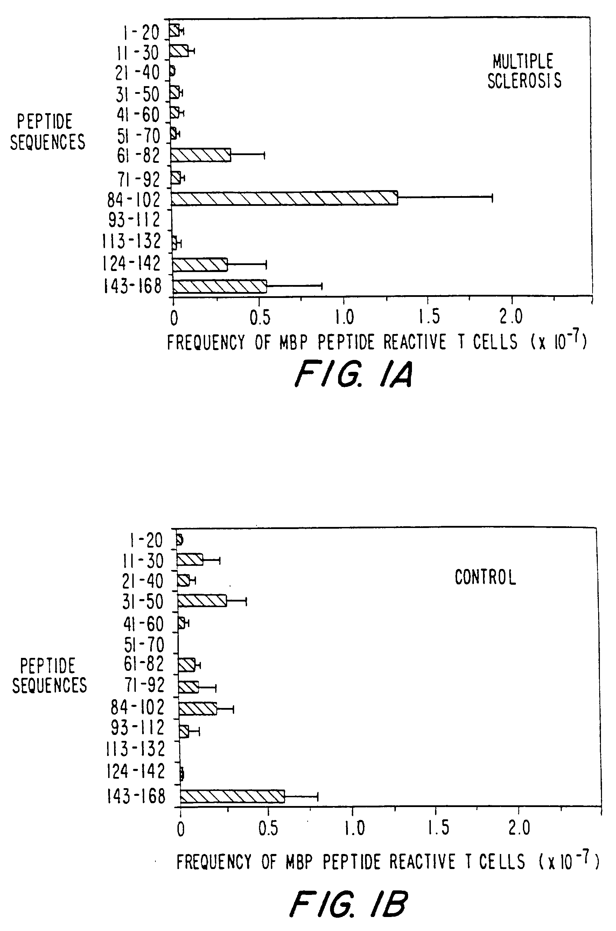

The frequency of MBP-peptide specific cell lines from normal subjects and other neurologic disease controls were virtually identical and thus combined for analysis. The mean frequency of T-cell lines from subjects with MS that were selectively reactive to MBP residues 84-102 was higher as compared with controls (Figure 1). Significant but less striking increases in reactivity to MBP residues 61-82 and 124-142 were also observed in MS patients, while both MS and control subjects showed high frequencies of T-cell lines reactive with MBP residues 143-168. IL-2 was used to select the T-cell lines that were activated. After primary stimulation with IL-2 the thus activated cell lines recognized MBP peptides.

Expansion of these findings into a larger population of MS patients was done, using the techniques described above. An additional 132 T-cell lines (63 from MS patients and 88 from normal controls) were studied, with the results reported in Table 3, below. In summary, these results support the conclusion of peptides containing MBP amino acid residues 84-102 as the immunodominant domains of MBP.

**TABLE 3**

| Subject | Stimulus | No. of MBP-Peptide Reactive Lines | Peptide Reactivity (Fraction of MBP-Reactive Lines) |
|---|---|---|---|
| MS-HY | MBP | 17 | 84-102 (17/17) |
| | IL-2 | 4 | 84-102 (4/4) |
| MS-SW | MBP | 8 | 84-102 (2/8) |
| | | | 143-158 (5/8) |
| | | | other (1/8) |
| | IL-2 | 3 | 143-168 (2/3) |
| | | | other (1/3) |
| MS-CY | MBP | 14 | 143-168 (9/14) |
| | | | other (5/14) |
| | IL-2 | 4 | 143-168 (3/4) |
| | | | other (1/4) |
| MS-HK | MBP | 7 | 143-168 (6/7) |
| | | | other (1/7) |
| | IL-2 | 6 | 143-168 (5/6) |
| | | | other (1/6) |
| MS-JA | MBP | 10 | 84-102 (1/10) |
| | | | 143-168 (8/10) |
| | IL-2 | 4 | 143-168 (4/4) |
| MS-MI | MBP | 5 | 84-102 (3/5) |
| | | | 143-168 (1/5) |
| | | | other (1/5) |
| | IL-2 | 2 | 84-102 (1/2) |
| | | | 143-168 (1/2) |
| MS-AN | MBP | 2 | 143-168 (2/2) |
| | IL-2 | 2 | 143-168 (2/2) |
| MS-ST | MBP | nd | nd |
| | IL-2 | 18 | 84-102 (6/18) |
| | | | 143-168 (6/18) |
| | | | 93-142 (3/18) |
| | | | other (3/18) |
| nd = not done | | | |
| NS-kw | MBP | 8 | 84-102 (2/8) |
| | | | 143-168 (5/8) |
| | | | other (1/8) |
| | IL-2 | 1 | |
| | | | 84-102 (100%) |
| NS-jl | MBP | 12 | 84-102 (10/12) |
| | | | other (2/12) |
| | IL-2 | 12 | 84-102 (7/12) |
| | | | 143-168 (5/12) |
| NS-aa | MBP | 2 | 84-102 (2/2) |
| | IL-2 | 2 | 84-102 (2/2) |
| NS-dd | MBP | 12 | 84-102 (4/12) |
| | | | 143-168 (6/12) |
| | | | other (2/12) |
| | IL-2 | 2 | 84-102 (1/2) |
| | | | 143-168 (1/2) |
| NS-nb | MBP | 37 | 84-102 (37/37) |
| | IL-2 | 2 | 84-102 (2/2) |

**TABLE 4**

| subject | CSF cells/nm³ | frequency of IL-2 responsive T-cells x 10⁻⁴ | | frequency of IL-2 responsive T-cells x 10⁻⁴ | |
|---|---|---|---|---|---|
| | | CSF | PBMC | CSF | PBMC |
| patients with MS | | (A) | (B) | (C) | (D) |
| MS-1 | 12.2 | 18.0(13.1-25.7)^{a} | 4.7(2.3-8.2) | 2.6(1.8-6.6) | N.D.^{b} |
| MS-2 | 1.5 | 10.4(6.1-14.7) | 6.7(4.2-11.8) | 5.5(4.3-9.2) | N.D. |
| MS-3 | 1.2 | 7.7 (4.3-12.8) | 4.7(2.9-8.8) | 3.8 (2.2-7.2) | N.D. |
| MS-4 | 1.2 | 16.6(11.5-23.7) | 13.3 (9.9-19.7) | 9.1 (6.9-14.8) | N.D. |
| MS-5 | 1.5 | 17.5 (13.1-24.2) | 11.2(7.8-17.2) | 8.3 (5.3-14.5) | N.D. |
| MS-6 | 2.0 | 23.3 (16.9-32.4) | 8.7 (6.4-13.2) | 20.0 (14.8-28.0) | N.D. |
| MS-7 | 3.4 | 25.0(18.7-33.1) | 7.7(4.2-12.4) | 8.4(6.0-13.5) | 1.8 (1.4-3.2) |
| MS-8 | 2.8 | 7.7 (4.3-11.9) | 2.3 (1.7-5.8) | 6.7 (4.2-11.8) | 1.9 (1.3-4.4) |
| MS-9 | 5.0 | 12.1 (8.6-17.8) | 12.1 (8.8-17.4) | 1.7 (1.2-4.3) | N.D. |
| MS-10 | 4.8 | 24.3 (17.2-32.2) | 2.4 (6.4-13.5) | 3.4 (2.3-7.4) | N.D. |
| MS-11 | 5.7 | 23.8 (16.8-32.5) | 3.2(2.2-7.8) | 4.9 (2.9-8.6) | N.D. |
| MS-12 | 4.6 | 6.1 (3.6-11.1) | 2.7 (1.8-5.6) | N.D. | N.D. |
| MS-13 | 2.2 | 17.8 (13.2-24.3) | 10.0 (1.8-15.4) | N.D. | N.D. |
| MS-14 | 3.4 | 25.0 (18.7-33.5) | 16.6 (11.5-23.5) | 2.4 (1.7-6.9) | N.D. |
| MS-15 | 3.8 | 18.2 (13.4-24.8) | 8.3 (5.8-11.4) | 6.6 (5.2-10.8) | N.D. |
| MS-16 | 4.5 | 6.2 (4.3-11.8) | 4.2(2.4-7.5) | 6.4 (5.2-10.7) | N.D. |
| MS-17 | 2.6 | 12.5 (8.6-18.3) | 6.2 (4.6-10.8) | 1.4 (1.3-4.5) | N.D. |
| MS-18 | 1.2 | 7.7 (4.2-13.1) | 4.3 (2.7-9.2) | N.D. | N.D. |
| MS-19 | 4.6 | 11.1 (7.6-17.0) | 2.0 (1.4-5.6) | 16.5 (11.5-24.1) | 2.7(1.8-5.2) |
| MS-20 | 3.8 | 23.8 (16.9-32.0) | 8.3 (6.0-12.9) | N.D. | N.D. |
| mean | 3.60 | 15.7 | 7.8 | 5.4 | 0.32 |

| patients with OND | | (E) | (F) | (G) | (H) |
|---|---|---|---|---|---|
| OND-1 | 5.0 | 10.4 (7.3-16.4) | 9.6(7.3-14.6) | N.D. | N.D. |
| OND-2 | 2.0 | 7.2 (5.1-10.8) | 8.3 (5.8-12.4) | N.D. | N.D. |
| OND-3 | 2.4 | 4.6 (2.8-9.0) | 7.8(4.2-14.5) | N.D. | N.D. |
| OND-4 | 3.0 | 5.8 (4.6-10.1) | 5.4 (4.2-9.9) | N.D. | N.D. |
| OND-5 | 5.0 | 4.1 (2.3-8.5) | 8.7 (6.4-13,4) | N.D. | N.D. |
| OND-6 | 4.4 | 4.8 (2.9-9.2) | 6.8 (5.2-10.2) | N.D. | N.D. |
| OND-7 | 1.2 | 3.3 (2.0-7.9) | 4.2 (2.4-8.9) | N.D. | N.D. |
| OND-8 | 2.0 | 2.1 (1.3-5.0) | 4.9 (3.8-9.1) | N.D. | N.D. |
| mean | 3.13 | 5.2 | 6.9 | -- | -- |
| column pair | (A)-(B) | (C)-(D) | (E)-(F) | (A)-(B) | (B)-(F) (C)-(G) |
| p value^{c} | 0.0001 | 0.0001 | 0.165 | 0.001 | 0.578 0.0001 |

| | | | | | |
|---|---|---|---|---|---|
| ^{a} 95% confidence limits. | | | | | |
| ^{b} not detectable at the cell concentration used. | | | | | |
| ^{c} calculated by t-test. | | | | | |

The foregoing results in Table 4 demonstrate that a dramati cally higher number of additional MBP-reactive T-cell clones can be identified in MS patients (compared to controls) upon primary stimulation of MBP-specific T-cells with IL-2. This indicates that MBP-reactive T-cell clones can be de-activated (possibly de-anergized) by exposure to IL-2, subsequent to which they become reactive to MBP peptides.

### EXAMPLE 2: MECHANISM OF INDUCTION OF IMMUNE TOLERANCE

The experiments in this Example were done to compare the effectiveness of suppression of EAE using different fragments of MBP, to compare oral and intravenous administration of the protein fragment, and to compare treatment of the disease state when it was induced or adoptively-transferred. Induced EAE occurs when MBP is used to immunize a host and is administered intramuscularly in conjunction with an adjuvant, while adoptively transferred disease occurs when an MBP-reactive cell line is activated then injected into the animal. (see Induction of EAE section below for details.) In this example, the following materials and methods were used.

**Animals.** Female Lewis rats 6-8 weeks of age were obtained from Harlan-Sprague Dawley Inc. (Indianapolis, IN). Animals were housed in Harvard Medical School Animal Care Facilities and maintained on standard laboratory chow and water ad libitum. Animals were maintained in accordance with the guidelines for the Committee on Care of Laboratory Animals of the Laboratory Research Council (Pub. #DHEW:NIH, 85-23, revised 1985).

**Antigens and Reagents.** Guinea pig MBP was purified from brain tissue by the modified method of Deibler et al. (Prep. Biochem. 2:139, 1972). Protein content and purity were checked by gel electrophoresis and amino acid analysis. Concanavalin A and histone were obtained from Sigma (St. Louis, MO). Peptides were synthesized in the peptide facility of the Center for Neurologic Disease, Brigham and Women's Hospital, and purified on HPLC. The amino acid sequences of the peptides synthesized are: 21-40, MDHARHGFLPRHRDTGILDS (immunosuppressive epitope region when orally administered to rats); 71-90, SLPQKSQRSQDENPVVHF (immunodominant encephalitogenic region in rats); 151-170, GTLSKIFKLGGRDSRS.

**Induction of Tolerance.** For oral tolerance or active suppression, rats were fed 1 mg of MBP dissolved in 1 ml PBS, or PBS alone, by gastric intubation with a 18-gauge stainless steel animal feeding needle (Thomas Scientific, Swedesboro, NJ). Animals were fed five times at intervals of 2-3 days with the last feeding two days before immunization. For intravenous tolerance or clonal anergy, rats were injected with 0.1 mg of MBP, MBP peptides, or histone dissolved in 0.1 ml PBS, or PBS alone. Animals were injected via the ocular vein five times at intervals of 2-3 days with the last injection two days before immunization.

Induction of EAE. For actively induced disease, Lewis rats were immunized in the left foot pad with 25 µg of guinea pig MBP in 50 µl of PBS emulsified in an equal volume of complete Freund's adjuvant (CFA) containing 4 mg/ml of mycobacterium tuberculosis (Difco). For adoptively transferred EAE, an MBP active T cell line was established from rats immunized with MBP in CFA, raised and maintained according to the method of Ben-Nun et al. (Euro. J. Immunol. 11:195, 1982). Encephalitogenic cells were collected after activation by culture with Concanavalin A (ConA) (2 µm/ml) using irradiated thymocytes from immunized animals as APCs. Cells were harvested from cultures via a ficol hypaque gradient (Hypaque 1077, Sigma) and washed twice in PBS prior to transfer. 5x10⁶ encephalitogenic cells were injected intraperitoneally in 0.1 ml PBS into irradiated (750 rads, 24 hrs. earlier), recipient rats. Cell viability of both modulator and encephalitogenic cells was determined by trypan blue exclusion and was greater than 90%. In all experiments 5 animals were used per experimental group.

**Purification of T cell subsets for adoptive transfer of protection following oral tolerization.** Depletion of lymphocyte subsets was performed by negative selection using magnetic beads according to a modified method of Cruikshank et al. (J. Immunol. 138:3817, 1987). Spleen cells were incubated with a 1:10 dilution of mouse anti-rat CD8 or CD4 monoclonal antibody (clones OX/8 or W3/25 respectively, Serotec/Bioproducts, Indianapolis, IN), for 30 minutes on ice, washed twice, and then added to prewashed magnetic particles, with an average diameter of 4.5 µm (M-450) with goat anti-mouse IgG covalently attached (Dynal, Fort Lee, NJ). The quantity of magnetic beads used was calculated as being 10 times the estimated target cell population. The cells were incubated with the beads in 0.5 ml of RPMI 1640 medium supplemented with 10% fetal calf serum in a 10 ml round bottom test tube (Nunc) for 30 min. on ice with gentle shaking every 5 min. After incubation, the bead/cell suspension was washed with 5 ml of medium, and the cell-mAb-bead complexes were separated from unlabeled cells in a strong magnetic field using a magnetic-particle concentrator (Dynal-MPC-1) for 2 minutes. The supernatant was removed, and the procedure was repeated twice to obtain the nonadherent fraction. The cells in the CD4⁺ and CD8⁺ depleted populations were >95%CD4⁺CD8⁻ or CD4⁻CD8⁺, as demonstrated by indirect flow cytometry. Whole spleen populations from MBP fed or control animals were cultured (5 x 10⁶ cells in 1 ml of proliferation media), in the presence of Con-A (2 µg/ml). Depleted populations were cultured at a concentration of 2.5 x 10⁶ cells per ml. The resulting subsets were used as modulator cells.

**Clinical evaluation.** Animals were evaluated in a blinded fashion every day for evidence of EAE. Clinical severity of EAE was scored as follows: 0, no disease; 1 limp tail; 2, hind limb paralysis; 3, hind limb paraplegia, incontinence; 4, tetraplegia; and 5 death. Duration of disease was measured by counting the total number of days from disease onset (usually days 10 or 11 after active immunization and 3-5 days after adoptive transfer of disease) until complete recovery for each animal.

**Delayed type hypersensitivity (DTH) testing.** DTH was tested by injecting 25 µg of MBP in PBS subcutaneously in the ear. Thickness was measured by a blinded observer, before and 48 hours after challenge, using micrometer calipers (Mitutoyo, Japan). The difference of ear thickness before and after challenge was recorded for each animal, and the result was expressed as the mean for each experimental group ± SEM.

**Histology.** Histologic analysis of pathological changes was performed in rats with adoptively transferred EAE. Spinal cords were removed on day 15 after adoptive transfer and fixed with 10% neutral buffered formalin. Paraffin sections were prepared and stained with Luxol fast blue-hematoxylin and eosin, by standard procedures (Sobel et al., J. Immunol. 132:2393, 1984). Spinal cord tissue was sampled in an identical manner for each animal and numbers of inflammatory foci per section (clusters of >20 or more aggregated inflammatory cells), in parenchyma and meninges were scored in a blinded fashion (Sobel et al., supra).

**Statistical analysis.** Clinical scales were analyzed with a two-tailed Wilcoxon rank sum test for score samples, chi square analysis was used in comparing the incidence of disease between groups, and comparison of means was performed by using the Student's t-test. For individual experiments, 5 animals were used per group.

### RESULTS

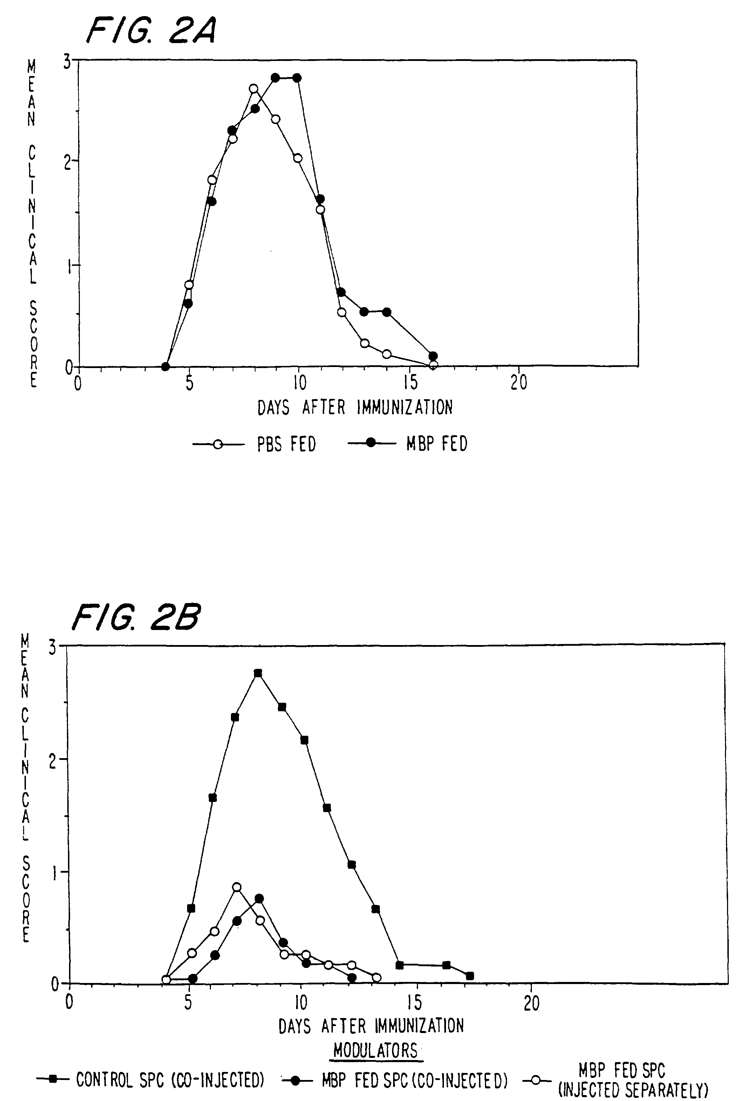

**Suppression of adoptively transferred EAE by oral tolerization to MBP.** To evaluate the effect of prior oral administration of MBP on adoptively transferred EAE, MBP-fed and control rats were intraperitoneally inoculated with 5x10⁶ MBP-specific, Con-A stimulated, encephalitogenic line cells. MBP reactive cells were transferred 2 days after the last feeding. Figure 2A graphs the clinical scores of animals which were orally tolerized to MBP and then inoculated with the MBP-specific cells (black circles) and compares them with the clinical scores of naive animals similarly inoculated (open circles). As shown in this figure, oral administration of MBP had no effect on adoptively transferred EAE. The present inventors propose that the failure of oral tolerization to suppress adoptively transferred EAE is due to the fact that the transplanted encephalitogenic T-cells are activated and able to migrate to the target organ rapidly wherein they initiate immune attack before sufficient numbers of suppressor T-cells: migrate to the lymph nodes; and (iii) migrate to the target organ (brain). Thus, the ratio of regulatory to encephalitogenic cells present at the target organ and the timing of their entry appear to be critical.

However, adoptively transferred EAE was suppressed when spleen cells from orally tolerized animals were co-transferred with the encephalitogenic cells to naive recipients (Figure 2B), indicating that already-elicited suppressor T-cells can successfully prevent disease even when they are co-administered with the immune attack cells.

Figure 2B graphs the clinical scores of animals which were co-injected with 5 x 10⁶ encephalitogenic cells with 1.5 x 10⁶ spleen cells from animals orally tolerized to MBP. For co-transfer, cells from orally tolerized animals were mixed with encephalitogenic cells and injected (black circles). As also shown in Figure 2B, similar protection was observed when encephalitogenic and modulator cells were injected separately in the right and left flanks (open circles), which indicates that the protective effect is not due to interaction between suppressor T-cells and attack T-cells. The clinical scores of positive control animals in Fig. 2B are indicated by black squares.

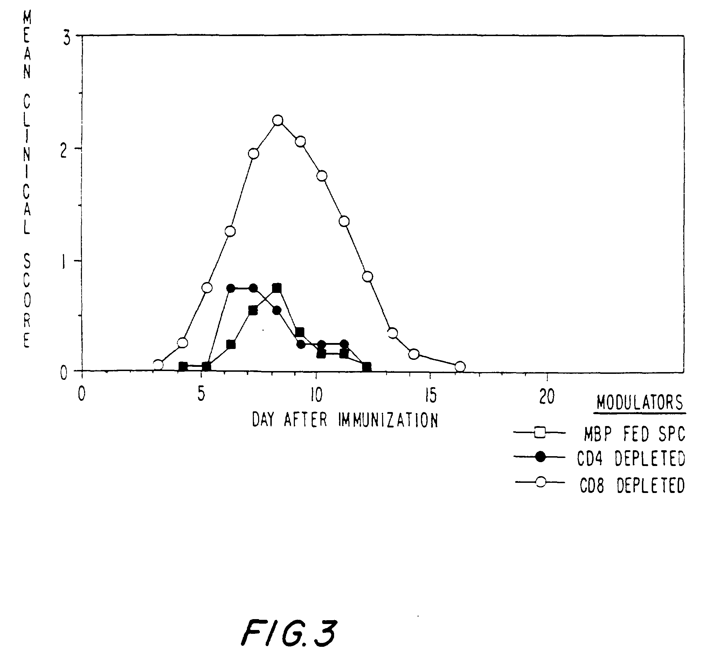

**Suppression of adoptively transferred EAE is dependent on CD8**^{**+**} **T cells from orally tolerized animals.** To determine whether suppression of adoptively transferred EAE was dependent on a specific T-cell subset, spleen cells from MBP fed animals were depleted on CD4⁺ or CD8⁺ T-cell subsets prior to adoptive transfer and used as modulators. As shown in Figure 3, adoptive transfer of protection was abrogated by transfer of CD8⁺ depleted spleen cells, but not by transfer of CD4⁺ depleted spleen cells (mean maximal score = 2.3±0.2 vs. 0.7±0.2, respectively, p<0.01). Open squares: unselected spleen cell population, black circles CD4⁺ depleted spleen cells; open circles CD8⁺ depleted spleen cells.

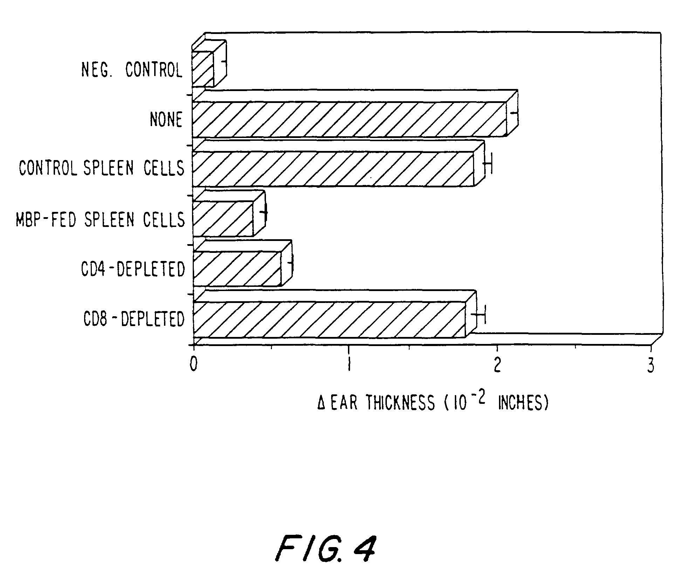

**Delayed type hypersensitivity (DIR) responses associated with adoptively transferred EAE.** A correlation between DTH responses and the suppression of actively induced EAE following oral tolerance has been found (Miller et al., J. Exp. Med. 174:791, 1991; Miller et al., Proc. Natl. Acad. Sci. 89:421, 1992). To determine whether a similar correlation existed in adoptively transferred EAE, DTH responses were measured. As shown in Figure 4, prominent DTH responses developed in animals undergoing adoptively transferred EAE and DTH responses were suppressed by the co-transfer of splenocytes from animals orally tolerized to MBP. The suppressed DTH responses were abrogated by depletion of CD8⁺, but not CD4⁺ T-cells prior to transfer. (Δ ear swelling CD4⁺ depleted vs. CD8⁺ depleted = 0.6±0.1 vs. 1.8±0.2, p<0.01).

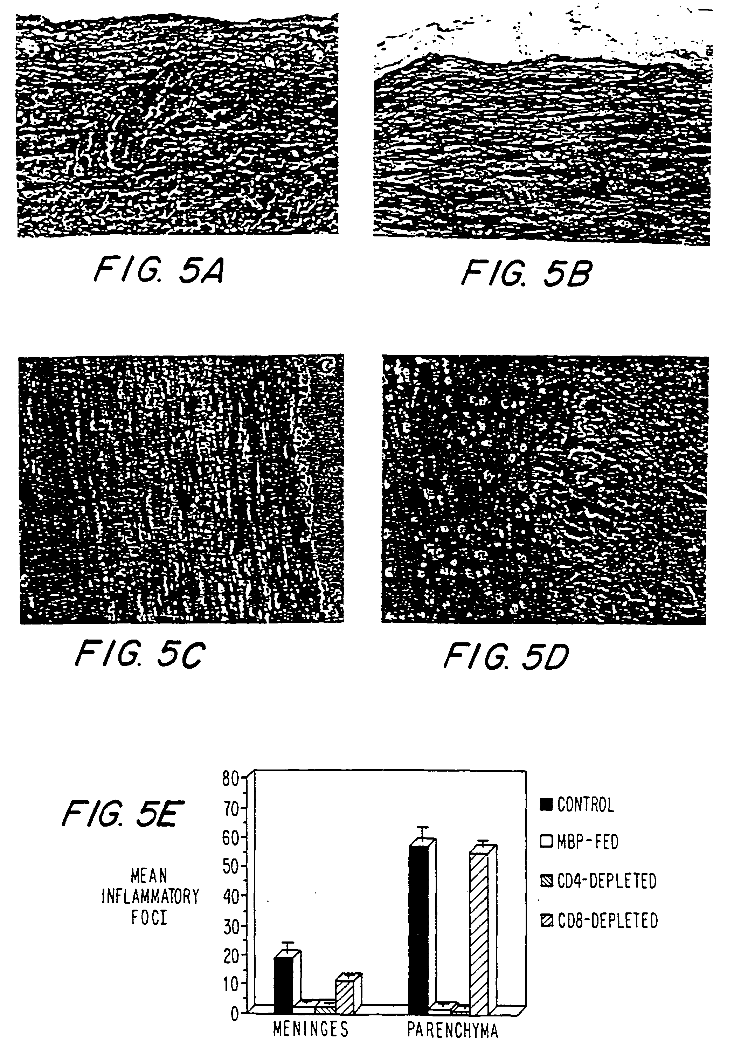

**Effect of co-transfer of cells from MBP orally tolerized animals on CNS histology in adoptively transferred EAE.** Oral administration of MBP suppresses CNS inflammation in actively induced EAE (Higgins et al., J. Immunol. 140:440, 1988). Nevertheless, not all immune specific immunomodulatory treatments of EAE that suppress clinical disease affect CNS inflammation (Offner et al., Science 251:430, 1991). As shown in Fig. 5, there was decreased inflammation in both the parenchyma and meninges when cells from MBP-fed animals were transferred and this suppression was observed when CD4⁺ depleted, but not CD8⁺ depleted modulator spleen cells from orally tolerized animals were transferred. Number of CNS (parenchyma + meninges) inflammatory foci for the specific groups were as follows: Control = 76±8.2; MBP fed = 3.8±1.8; CD4⁺ depleted = 2.8±1.0; CD8⁺ depleted = 65±4; (p<0.01, MBP fed and CD4⁺ depleted vs. control or CD8⁺ depleted).

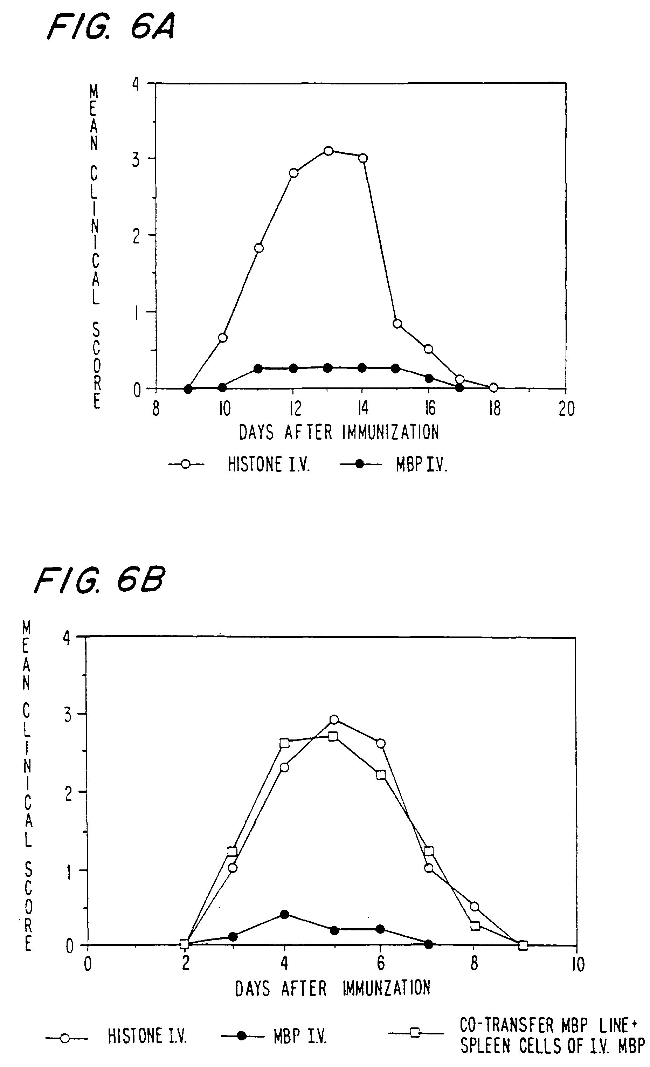

**Suppression of actively induced and adoptively transferred EAE following IV administration of MBP.** As shown in Figure 6A, intravenous (IV) injection of MBP markedly suppressed EAE actively induced by immunization with MBP/CFA (mean maximal score = 0.5±0.2, vs. control injected histone = 3.0±0.3; p<0.01), in an analogous manner to suppression by oral tolerization with MBP. In contrast to oral tolerization, however, which did not protect against adoptively transferred EAE (Figure 2A), IV injection of MBP also suppressed adoptively transferred EAE (mean maximal score = 0.4±0.2, vs. control = 3.2±0.2; p<0.01). (Figure 6B) However, unlike oral tolerization, disease protection could not be adoptively transferred with spleen cells from IV tolerized animals when such cells were co-transferred with an MBP encephalitogenic line (mean maximal score = 2.8±0.2, vs. control p = N.S. (Figure 6B).

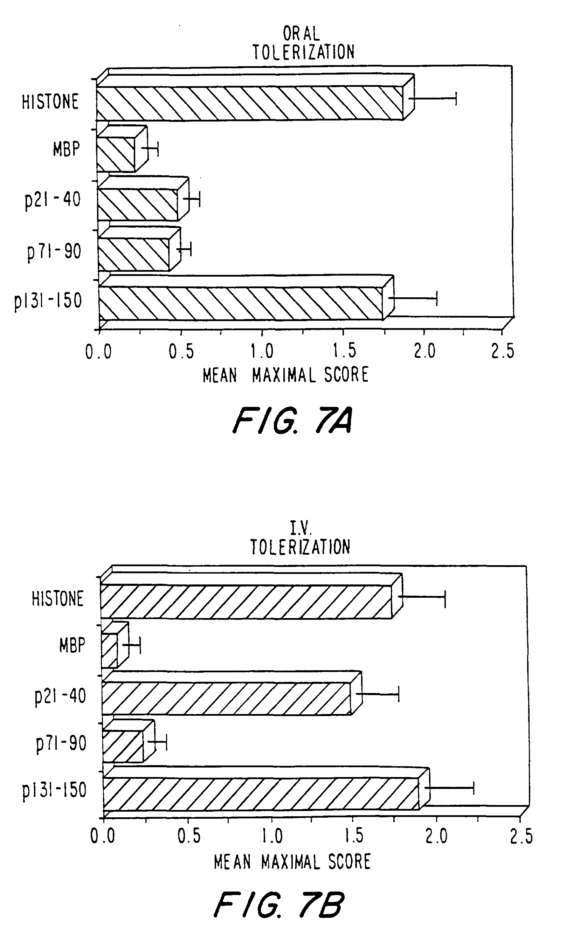

**Suppression of EAE following oral or IV administration of MBP peptides.** To further investigate the mechanism of oral vs. IV tolerance, MBP peptides encompassing both encephalitogenic and non-encephalitogenic regions of MBP were administered both orally and intravenously prior to immunization for actively induced disease. MBP peptide 71-90 of guinea pig MBP is encephalitogenic in Lewis rats (Swanborg et al., J. Immunol. 114:191, 1975). As shown in Figure 7, suppression of EAE via IV tolerization only occurred with whole MBP and encephalitogenic peptide 71-90, but not with guinea pig MBP peptide 21-40. Oral tolerization with 21-40, however, was effective in suppressing EAE. Guinea-pig peptide 21-40 was chosen as experiments demonstrated that it triggered TGF-β release from spleen cells of rats orally tolerized to whole MBP. Miller, A. et al. FASEB 6:1686, 1992. Control guinea pig MBP peptide 131-150 did not suppress when administered either orally or intravenously. Of note is that in addition to suppressing via the IV route, encephalitogenic MBP peptide 71-90 also suppressed when given orally. This result indicates that peptides derived from the immunodominant domain of a given MBP towards a given host can suppress T-cell function when they are orally or intravenously administrated, but do so by different mechanisms depending on the route and protocol of administration.

The results of these experiments show that there are basic differences in the mechanism of suppression of EAE between orally and parenterally (e.g. intravenously) administered MBP. The results suggest that orally administered antigen acts predominantly via the generation of active suppression, whereas parenterally administered antigen acts via clonal anergy. Specifically supporting this conclusion is the inability of spleen cells from IV tolerized animals to suppress adoptively-transferred EAE. Additionally, different fragments of MBP were more or less effective in suppression with the different routes of administration. (see, for example, Figure 7) The present findings may be used to advantage in designing immunosuppressive methods based on antigen-driven tolerance, such as the method of the present invention.

### EXAMPLE 3: FINE-TUNING OF THE DETERMINATION OF THE IMMUNODOMINANT EPITOPE OF HUMAN MBP IN HUMANS BY ASSESSING ABILITY OF OVERLAPPING PEPTIDES TO STIMULATE MBP SPECIFIC T-CELL CLONES

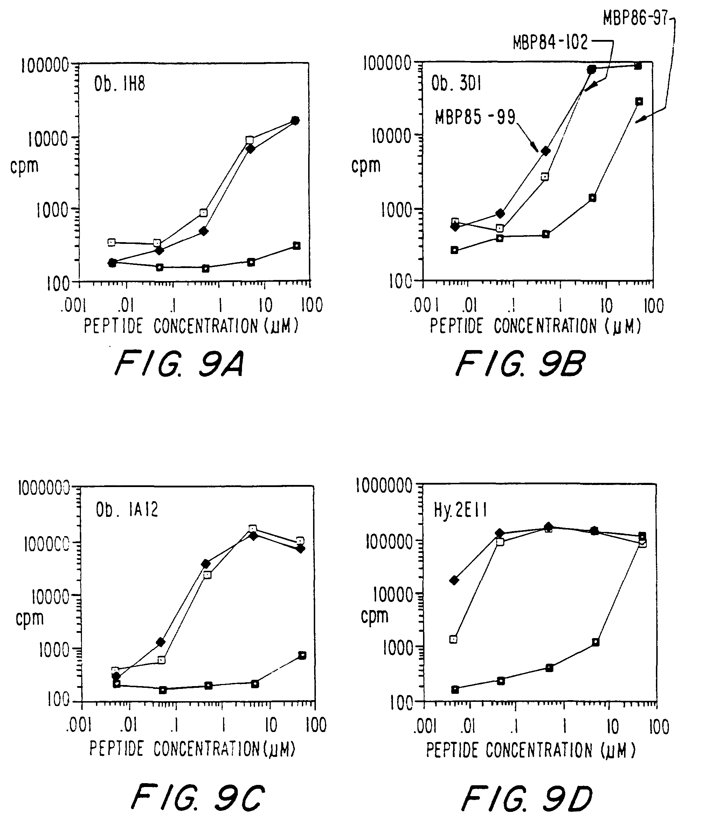

Using the cell proliferation assay described in Example 1 above, the ability of a peptide consisting of the immunodominant domain of human MBP (i.e. the sequence of amino acids 84-102) to stimulate T-cell proliferation was assessed and compared to that of a series of peptides having amino acid sequences representing N-terminal and C-terminal progressive truncations of the immunodominant domain was determined. As shown in Figure 8, (and Table 5 below) the tested T-cell lines proliferated with a N-terminal truncation down to amino acid 85, after which there was a dramatic decrease in ability of the fragment to activate proliferation. Progressive C-terminal truncation quickly drastically affects stimulation ability, where truncation to only residue 99 does not substantially affect epitope function. In absence of C-terminal truncation, as shown in Figure 8, loss of amino acids 85 and 86 also appear to be tolerated by the tested clone. In Figure 9, four different T-cell clones were exposed to whole MBP 84-102 peptide and to peptides 85-99 and 86-97 to test whether different peptides caused differences in proliferation of the different clones. Although individual clones have inherently different abilities to proliferate in the presence of an epitopic peptide, nevertheless, the ability of a particular peptide to cause a clone to proliferate is qualitatively similar from clone to clone. From these studies it appears that the fragment amino acids 85-99 would comprise a minimal immunodominant fragment able to stimulate T-cell activity for all T-cell clones.

Table 5, below lists the ability of human MBP (84-102) specific T-cell clones to proliferate in the presence of MBP (84-102) peptide and truncated or modified versions of this peptide. The numbers in the matrix are peptide concentrations (expressed in micromolar) that give 50% maximal stimulation of the T-cell clones. Maximal stimulation of the T-cell clones was assessed by exposing them to unmodified untruncated MBP (84-102) peptide. The bold face designates "50" and ">50" mean a five-fold or more than a five-fold loss in stimulative activity compared to the untruncated unmodified MBP (84-102) peptide.

**TABLE 5**

| **Comparison of the Relative Efficiency of Truncated Peptides to Stimulate MBP (84-102) Specific T cell clones** | | | | | | | | |
|---|---|---|---|---|---|---|---|---|
| | Ob.1H8 | Ob.1- | Ob.2G9 | Ob.1C3 | Ob.1- | Ob.2F3 | Ob.3D1 | Hy.2- |
| | | E10 | | | A12 | | | E11 |
| 84-102 | 3.1 | 4.0 | 3.8 | 2.9 | 1.6 | 2.1 | 2.0 | 0.26 |
| 84- | 2.0 | 3.1 | 3.2 | 2.9 | 1.6 | 1.5 | 2.0 | 0.26 |
| 102tyr | | | | | | | | |
| 85-102 | 12 | 1.8 | 3.6 | 4.0 | 0.4 | 1.5 | 2.0 | 0.31 |
| 86-102 | >50* | >50 | >50 | 50 | 4.8 | 17 | 2.0 | 0.45 |
| 87-102 | | | | >50 | >50 | >50 | 4.2 | 0.80 |
| 88-102 | | | | | | | >50 | 50 |
| 89-102 | | | | | | | | >50 |
| 84-100 | 2.2 | 1.8 | 1.2 | 2.2 | 0.65 | 1.9 | 2.5 | 0.08 |
| 84-99 | 3.1 | 4.0 | 2.1 | 2.3 | 1.6 | 2.1 | 2.2 | 0.26 |
| 84-97 | >50 | >50 | >50 | >50 | 22 | 22 | 11 | 25 |
| 84-96 | | | | | >50 | >50 | 50 | >50 |
| 84-95 | | | | | | | >50 | |

Peptide concentrations (µM) that give 50% maximal stimulation (reference point: MBP(84-102) peptide) are given. *More than a fivefold loss in activity compared to the MBP(84-102) peptide.

### Example 4: T-CELL RECOGNITION OF THE ALANINE ANALOG PEPTIDES OF MBP 85-99-REACTIVE T-CELL CLONES

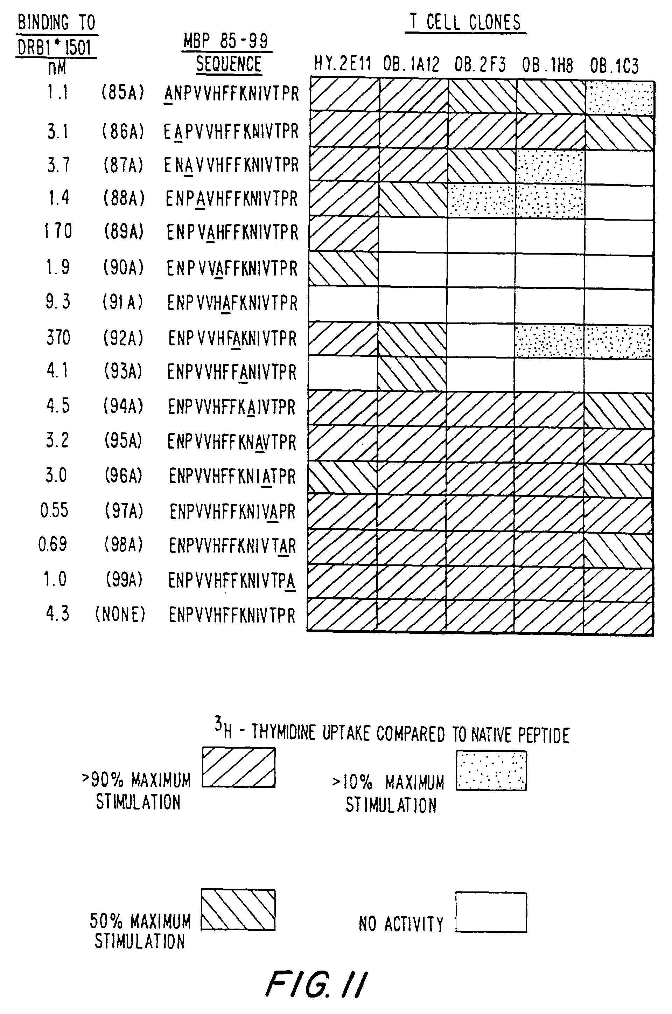

Fig. 11 shows the binding of each peptide to MHC (left column) and to a T-cell clone (right panel). DRB1*1501 transfected L cells present the immunodominant MBP (84-102) peptide to autoreactive T-cells. APC's were pulsed with the MPB (84-102) peptide (100µg/ml) for B-cell lines and 50 µg/ml for DR transfectants, irradiated with 5000 rad and co-cultured with the T-cell clones for three days followed by a thymidine pulse. The results (³H-thymidine uptake compared to native peptide) are expressed in black rectangles (90% of the maximum stimulation) grey with crosses (>50% of the maximum stimulation) light grey (>10% of the maximum stimulation) and white (no activity). The results show that the Val at position 898 and the Phe at position 92 are involved in MHC binding. Truncation data suggest that the isoleucine at position 95 is also involved in MHC binding. T-cell receptor contact points include His at 90, Phe at 91 and Lys at 93.

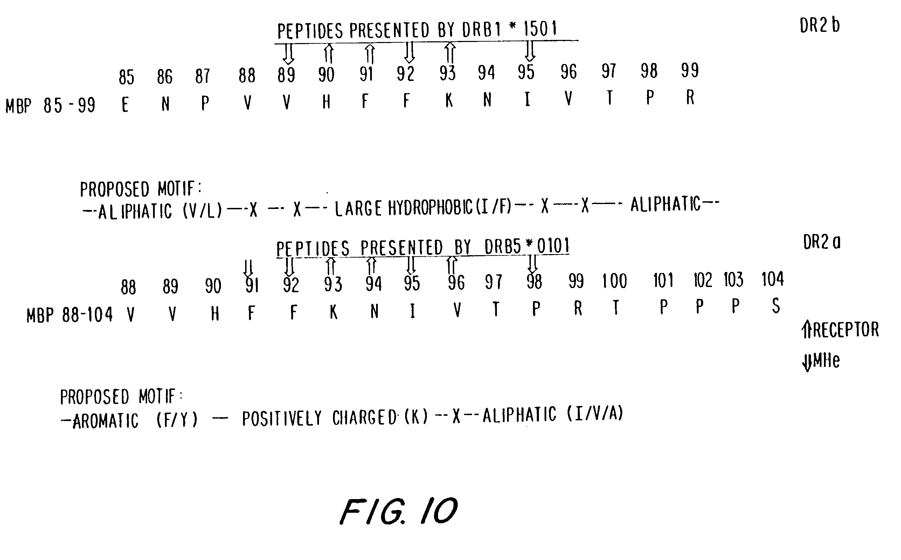

T-cell clone Hy.2Ell will tolerate an Arg substitution in place of Lys at 93 but the Ob T-cell clones will not. The proposed binding motifs for DRB1*1501 and DRB5*0101 are in Fig. 10. The arrows up indicate binding to the DR receptor and the arrows down indicate binding to the MHC of APC.

A series of analog peptides will be synthesized to both conservative and non-conservative amino acid substitutions at positions 88-95 that will be used. For example, the negative charge aspartic acid will be substituted with another negatively charged residue (glutomic acid), an amino acid with the same bulk but no charge (asparginine), an amino acid with the opposite charge (lys), and lastly a small amino acid such as ala. Proximately 60 peptides will need to be synthesized.

Preliminary data suggest that the 148-162 region of MBP is the minimal epitope for dominant epitope 143-168. When the core region of this peptide is defined using the methods outlined in Examples 2 and 3, a similar analysis as outlined in this Example 4 for MBP peptides 85-99.

Analog peptides will be analyzed for DR26- (DRB1*1501), DR2a-(DRB5*0101), DR4, DR7, and DQ1.1 binding. This analysis will elucidate further which amino acid residues are involved in MHC binding. Analog peptides that have no less than 10-times the binding capacity of the native peptide will be used to examine T-cell receptor specificity.

### EXAMPLE 5: TECHNIQUES

MBP was extracted from human brain tissue and purified on a CM-52 column using the highest molecular weight peak (18kD) as described (Chou, F.C.-H. et al J. Biol. Chem. 251: 2671, 1976). MBP peptides were synthesized using a solid phase method and were obtained from a commercial laboratory (Biosearch Lab Inc., San Raphael, CA) and were purified by high pressure liquid chromatography. The MBP peptide fragments used are set forth below in Table 6.

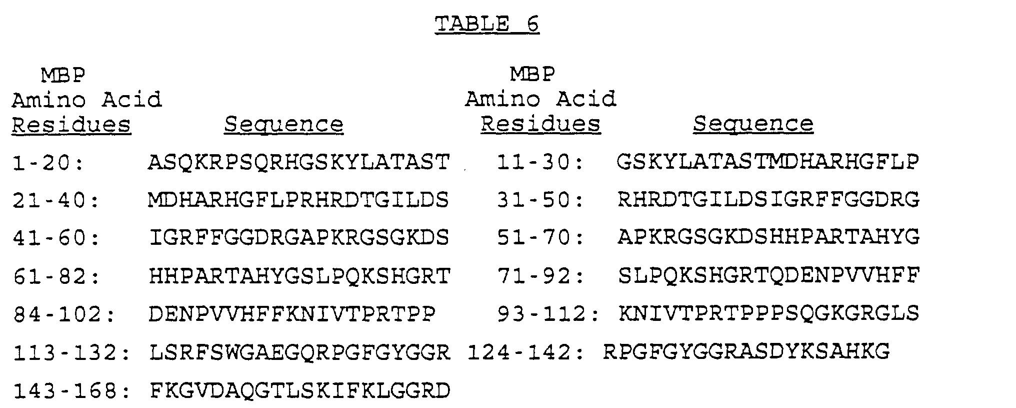

T-cell receptor TCR VB gene usage was determined by polymerase chain reaction (PCR) amplification using a panel of TCR VB primers followed by Southern blotting. T-cell lines were established from peripheral blood mononuclear cells by two rounds of stimulation with MBP followed by stimulation with an immunodominant human MBP peptide (amino acid residues 84-102), immunodominance of which had been determined by proliferation assays (as described in Example 6) using the Table 6 panel of 13 overlapping MBP peptides. Following a third round of stimulation with their specific MBP peptide, RNA was extracted from MBP-reactive T-cell culture pellets (20,000-50,000 cells) by extraction with guanidium-isothiocyanate/phenol-chloroform and isopropanol precipitation in the presence of carrier tRNA. Single-stranded cDNAs were synthesized using oligo-dT and AMV-reverse transcriptase (both available commercially from Bethesda Research Laboratories, Gaithersburg, MD). PCR (polymerase chain reaction as disclosed in U.S. Patent Nos. 4,800,159 issued January 24, 1989; 4,683195 issued July 28, 1987; and 4,683,202 issued July 28, 1987) amplification was performed using a panel of 19 oligonucleotides (specific for published TCR VB families --VB 1-20, Table 7) corresponding to the CDR2 region of the TCR B-chain and a CB (constant region of B chain) primer (Table 7) (as disclosed in Tilinghast, J.P. et al., Science 233: 879, 1986; Concannon, P. et al., Proc. Natl. Acad. Sci. 83: 6598, 1986; Kimura, N. et al., J. Exp. Med. 164: 739, 1986; Toyonaga, B. et al. Proc. Natl. Acad. Sci. 82: 8624, 1985; Kimura, N. et al., Eur. J. Immunol. 17: 375, 1987). Amplifications were done for thirty cycles (94°C 1 min., 55°C 2 min., 72°C 3 min.) using 1 microgram of each primer in 50 microliter reactions. Amplified products were separated in 1% agarose gels, transferred to nitrocellulose and Southern blots were hybridized with an internal oligonucleotide TCR-CB probe (Table 7). Probes were endlabeled with ³²P gamma-ATP and T4 polynucleotide kinase (Bethesda Research Labs.) to a specific activity of 10⁸cpm/ug and hybridized in 6xSSC/5xDenhardt's/0.05% pyrophosphate/100ug/ml denatured DNA/0.5% SDS for 18 hours at 37°C. Blots were washed at a final stringency of 6xSSC/70°C and autoradiographed for 2-18 hours. T-cell lines that were positive for more than two VB segments were considered not to be derived from a single MBP reactive T-cell and therefore excluded from analysis.

For sequencing, amplifications of cDNAs were performed with a VB17 primer (Table 7) specific for the leader segment containing an internal Pst I restriction site. Amplified DNA was treated with proteinase K, phenol/chloroform extracted, ethanol precipitated and digested with restriction endonucleases Bgl II and Pst I (available commercially, e.g., from Bethesda Research Labs., supra). Gel-purified DNA was ligated into M13 mp18 and single-stranded DNA was sequenced by the dideoxy-method (Sanger, F., et al., 1977, Proc. Nat'l. Acad. Sci., 74:5463). Negative controls were included during the procedure to test for possible contamination of RNA samples or reagents used for cDNA synthesis and amplification. The VB, CB and JB2.1 primer sequences used are set forth below in Table 7.

Amplified and non-amplified samples were handled separately, reagents were aliquoted and tested for the presence of amplified material and negative controls were included for different experimental steps (RNA isolation, cDNA synthesis, PCR amplification).

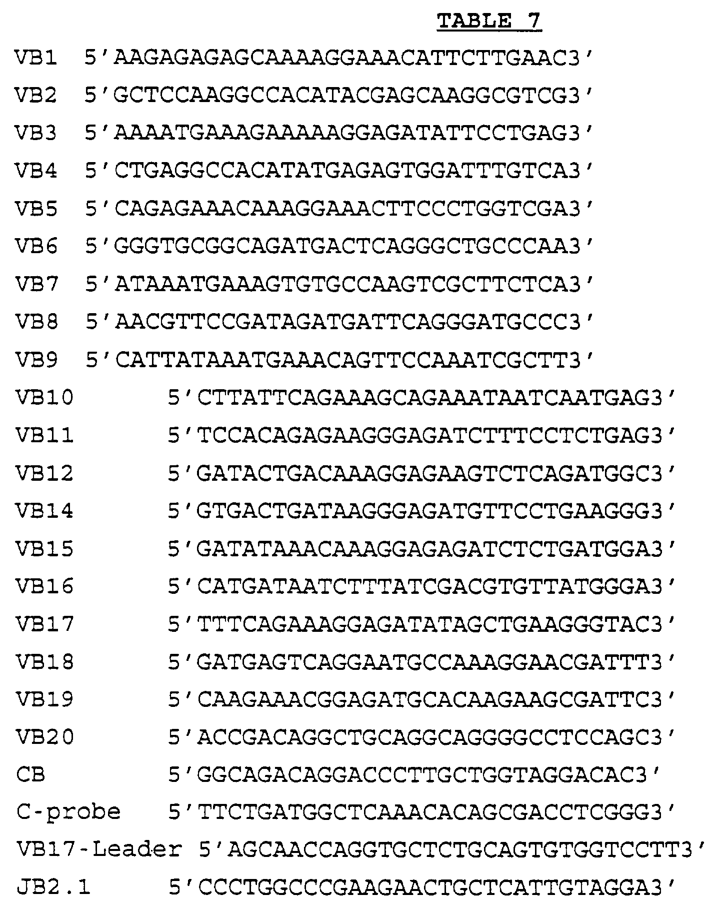

### EXAMPLE 6: IDENTIFICATION OF VB GENE USAGE IN T-CELLS ISOLATED FROM MS PATIENTS

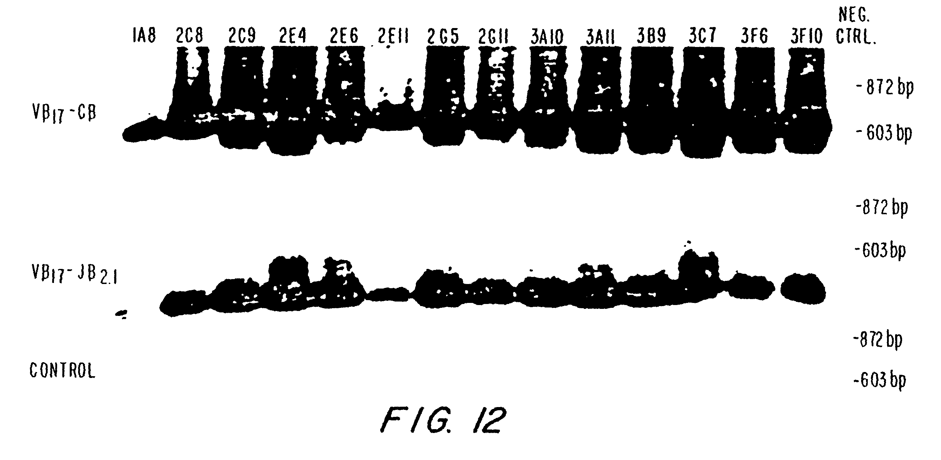

Two series of experiments were performed to test the validity of the above-described approach. First, it was demonstrated that all Table 7 primers except VB20 were able to amplify cDNA from peripheral blood T-cells (Figure 12). Secondly, the specificity of PCR amplifications was examined by analysis of VB gene usage in 69 independent T-cell clones previously established by single cell cloning with mitogen (such as phytohemagglutin, -- "PHA" -- and interleukin-2). Due to the high cloning efficiencies obtained, these clones provided a representative analysis of VB gene usage among peripheral blood T-cells. TCR VB gene usage could be determined for 65/69 (94.2%) of these T-cell clones indicating that a large proportion of the TCR VB repertoire was covered by the VB primers. While 58 of these clones (84%) were positive for a single VB, 7 clones (10.1%) were double-positive, possibly due to the presence of two rearranged and expressed TCR VB genes.

The TCR VB gene usage was then analyzed in sixty-five MBP-specific T-cell lines established from five patients with clinically-defined relapsing-remitting MS. Representative Southern blots from MBP reactive T-cell lines are shown in Figure 12 and VB genes usage for all cell lines analyzed are set forth in Table 8 below.

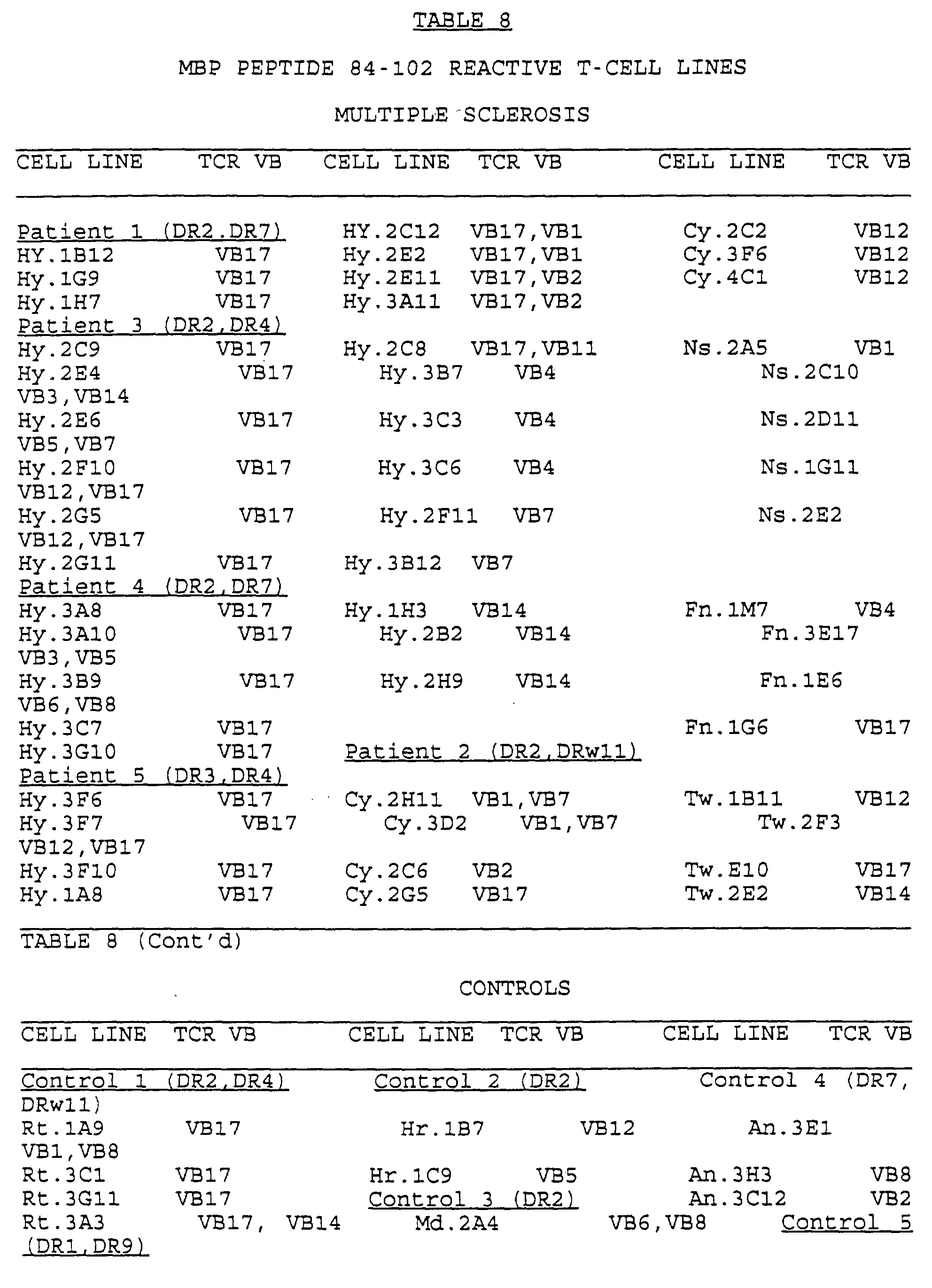

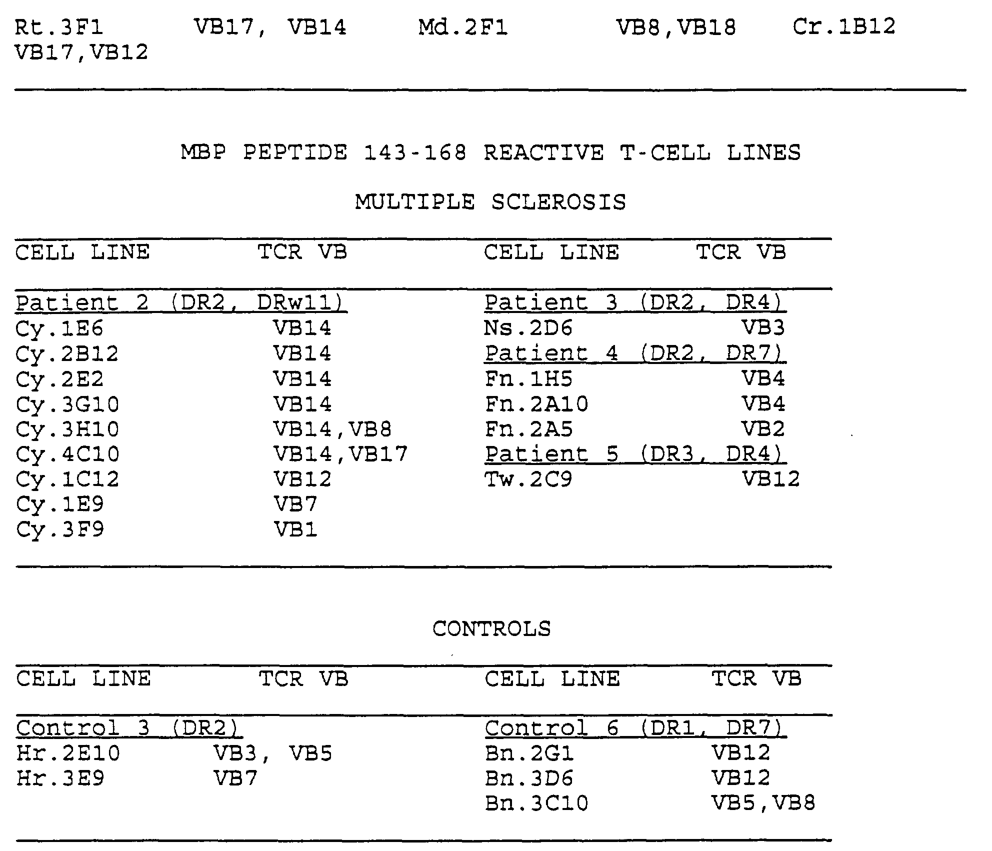

Fifty-one of these lines reacted with MBP residues 84-102, while fourteen T-cell lines were specific for MBP residues 143-168. Thirty-one MBP T-cell lines reactive to MBP amino acid residues 84-102 were analyzed from MS patient Hy (patient 1, Table 8). Twenty-three of these T-cell lines (74%) were found to use the VB17 gene segment, while eight other cell lines were restricted by either VB2, VB7 or VB14 gene segments. These results indicate that VB17 is the major recognition element in T-cell lines from this MS patient reactive with MBP residues 84-102. VB17 usage was also found among 6/20 T-cell lines examined from four other patients (patients 2-5, Table 8). The second TCR VB that was used by T-cell lines among these four patients was VB12 which was found in 7/20 T-cell lines reactive with MBP residues 84-102 (Table 8, Figure 12). This VB happens to be homologous to the mouse VB8.2 which is the predominant TCR used among encephalitogenic T-cells in mice and rats (Burns, F.R. et al., J. Exp. Med. 169: 27, 1989).

MS patient Cy expressed both the DR2 and DRw11 antigens and thus had T-cells that recognized either the immunodominant MBP region (84-102 residues) or the MBP 143-168 residues. This provided the opportunity to compare TCR VB usage among T-cells reacting to different MBP determinants (Figure 12). Of seven lines proliferating to MBP residues 84-102, three expressed VB12 and one expressed VB17 (Table 8). In contrast, 6/9 T-cell lines recognizing the MBP residues 143-168 used VB14 and only one line each used the TCR VB12 and VB17 TCR genes (Table 8). Southern blot analysis of five T-cell lines reactive with MBP residues 84-102 (VB12: Cy.2C2, Cy.3F6) or MBP residues 143-168 (VB14:Cy.1E6, Cy.2B12, Cy.2E2) are shown in Figure 12.

While VB12/VB13 is relatively common among normal peripheral blood T-cells (approximately 18%), VB17 is significantly less frequent (approximately 3%), as assessed by quantitative PCR. In contrast, VB17 was found in 34/63 (53.9%) of T-cell lines reactive with MBP residues 84-102, while it was only present in 3/32 (9.4%) of TCR VB genes in random mitogen derived T-cell clones obtained by single-cell cloning from a normal individual (Moretta, A. et al., J. Epp. Med. 157: 743, 1983; Hafler, D. A., et al., J. Exp. Med. 167: 1313, 1988). These data indicate that the VB17 TCR is selectively involved in the recognition of the immunodominant MBP 84-102 region.

In order to show that the TCR gene segment identified by PCR was the VB encoding gene used to recognize the MBP peptide, two VB17 positive T-cell lines (Hy.2H9 and Hy.2G5) were cloned by limiting dilution (Moretta, supra.). 11/11 individual clones established from these two cell lines, which were reactive with both MBP and MBP residues 84-102, were VB17+. Three of these clones were further analyzed using the complete panel of VB primers and were all found to be negative for the other VB segments.

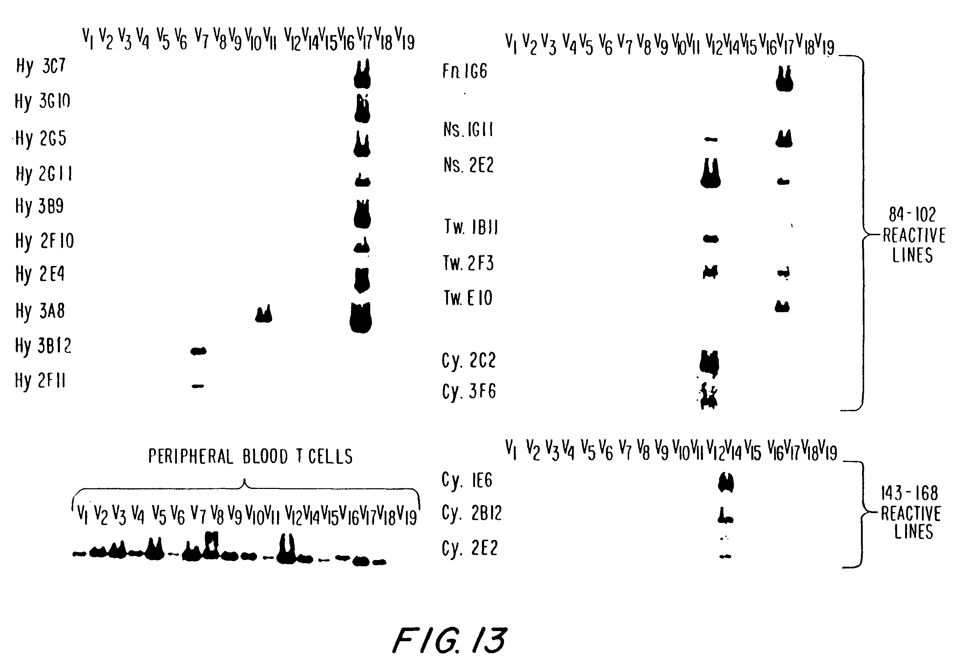

The VB sequences of four T-cell lines from patient Hy were found to be 100% homologous to the published VB17 sequence (as disclosed in Kimura, N., et al., Eur. J. Immunol. 17: 375, 1987). This sequence analysis confirms that specific VB segments were indeed amplified using this approach. Analysis of the VDJ (diversity-junctional) sequence indicated that all four of these T-cells used the same junctional JB2.1 segment and that 3/4 of them had the same VDJ sequence (Table 7). To determine how frequently the JB2.1 gene segment was used by VB17+ T-cells, the DNAs from 20 cell lines from MS patient Hy were amplified using the VB17 primer combination with a CB primer or a JB2.1 primer (Figure 13). All of these lines were found to be positive for VB17 as well as JB2.1 gene segments, while the negative controls (RNA extracted from all cell lines and not converted to cDNA, and reagents used for cDNA synthesis and amplification) were negative by PCR and Southern blotting. These data show a strong selection for the VB17-JB2.1 sequence elements in with MBP residues 84-102 reactive T-cell lines derived from patient Hy.

Two other T-cell lines using the VB17 TCR identified by PCR analysis and recognizing MBP residues 84-102 from MS patients Fn and Ns were sequenced and compared to sequences of TCR VB from MS patient Hy (Table 8). While the VB17 gene segment sequence was identical among T-cells reactive MBP residues 84-102 from the three patients, different JB sequence elements were found. Three results show a shared VB gene usage in T-cells recognizing an immunodominant MBP peptide between different individuals. In contrast, shared JB gene segment usage was found among T-cells derived from the same individual but not between different individuals.

Four of the five patients studied were positive for the disease-associated DR2 allele, while patient Tw was HLA-DR3, DR4. Nevertheless, three VB12/VB17 restricted cell lines were present among four lines analyzed from this MS patient (Table 8), indicating that shared MHC Class II antigens may not be mandatory for shared TCR VB gene usage with respect to recognition of MBP peptide 84-102.

### EXAMPLE 7: IDENTIFICATION OF THE MAJOR IMMUNODOMINANT REGION OF HUMAN MBP

A rapid T-cell cloning technique was used to examine whether there were immunodominant epitopes on human MBP reactive with Class II MHC phenotypes and the frequency of such reactivity. A total of 15,824 short term T-cell lines were generated from 51 subjects by culturing peripheral blood mononuclear cells (PMN) with purified MBP (obtained as in Example 5 above) followed 3 days later, and then every 3-4 days, by the addition of Interleukin-2 (IL-2) and Interleukin-4 (IL-4) (Genzyme, Boston, MA). On Day 13 of culture, an aliquot from each line was tested for reactivity to MBP. Lines reactive to MBP were then tested for reactivity to overlapping oligopeptide 20-mers encompassing the human MBP sequence as shown in Table 6 above. For MHC restriction experiments, lines reactive to an MBP peptide were restimulated for two more cycles, first with MBP and then with the specific MBP fragment recognized by that line. In a subgroup of patients, the frequency of T-cells recognizing proteolipid protein (PLP), another major encephalitogenic central nervous system antigen, was investigated.

MBP and PLP frequency analysis was performed on patients with definite, relapsing-remitting MS (as diagnosed by Magnetic Resonance Imaging -- "MRI" -- and clinical examination), as well as on subjects with other neurologic diseases and normal subjects (all age and sex matched to the MS patients).

The results are shown in Table 8A below.

**TABLE 8A**

| | | SEX(%) | MHC(%) | | | | #Ag REACTIVE LINES/ TOTAL # LINES | | MEAN FREQUENCY OF Ag REACTIVE LINES (%) | |
|---|---|---|---|---|---|---|---|---|---|---|
| | AGE | (M/F) | DR2 | DR4 | DRw11 | DQw1 | MBP | PLP | MBP | PLP |
| MULTIPLE SCLEROSIS (n=23) | 34.2+1.4 | 35/65 | 60.9 | 26.1 | 13.0 | 78.2 | 554/7746 | 20/432 | 7.18±2.38 | 3.34±1.56 |
| OTHER NEUROLOGIC | | | | | | | | | | |
| DISEASE (n=10) | 38.7±3.2 | 43/57 | 14.3 | 0.0 | 42.9 | 85.7 | 118/2880 | 3/384 | 4.10±1.04 | 0.90±0.62 |
| NORMAL (n=6) | 30.3±1.5 | 50/50 | 16.7 | 0.0 | 50.0 | 66.6 | 73/1742 | ND | 4.70±1.58 | ND |
| DR2+ CONTROLS (n=6) | 32.0±2.9 | 50/50 | 100 | 16.7 | 0.0 | 100 | 53/1728 | ND | 3.08±2.06 | ND |

Patients with MS were caucasian and had well- characterized relapsing remitting disease with at least two exacer-bations within the previous 24 months and positive lesions on Magnetic Resonance Imaging (MRI) at the time of blood drawing. Subjects with other central nervous system diseases had the following diagnoses: 1-3 weeks after either cerebrovascular accident [4] or brain trauma with CNS hemorrhage [4]; metastatic brain tumor [2]. The total number of T-cell lines reactive with either MBP or PLP and the total number of T-cell lines generated are shown in Table 8A ("Ag" means "antigen"). In addition, the frequencies of MBP- and PLP- reactive lines were calculated separately for each subject by dividing the number of MBP-reactive lines by the total number of lines generated and the mean value ± SEM are given.

While the frequency of MBP reactive lines was slightly higher in subjects with MS as compared to the other subjects, this was not statistically significant. There was more reactivity to PLP in patients with MS as compared to subjects with other neurologic diseases, but this did also not reach statistical significance.

Of a total of 302 cell lines from patients with MS that could be expanded and confirmed to react with MBP on repeated analysis, 140 (46.4%) reacted with MBP residues 84-102. In the control groups, 11 of a total of 100 MBP reactive T-cell lines (11.0%) recognized this MBP peptide. The actual frequency of T-cells derived from the peripheral blood that reacted with each MBP peptide for each individual subject was calculated. The mean values for patients with MS and the control subjects are shown in the next-to-rightmost column of Table 8A.

50,000 T-line cells were plated in triplicate with 50,000 irradiated APC, MNC (mononuclear cells) (Hafler, D. A., et al., J. Exp. Med. 167: 1313, 1988) for 72 hours in round bottom 96-well microtiter plates and wells were pulsed with [³H]-thymidine for the last 18 hours of culture. APC MNC were either cultured alone, pulsed with 100 micrograms/ml of synthetic MBP peptide 84-102, (determined to be the optimal concentration of peptide to induce proliferation), or pulsed with 100 micrograms/-ml of MBP. The average counts per minute (CPM) values for triplicate wells are shown in Table 9. DR and DQw haplotypes are given and haplotypes common with the patient (top line), who was positive for DR2, DR7, DQw1, DQw3, are underlined.

Proliferation of T-cell lines using a panel of different mononuclear cells (MNC) as antigen presenting cells (APC) are shown. Five T-cell lines reactive to MBP amino acid residues 84-102 from subject Hy were expanded by repeated cycles of stimulation with autologous irradiated MNC, pulsed with synthetic MBP peptide 84-102 and examined for recognition of this region of MBP.

For these studies, the panel of five T cell lines reactive with MPB residues 84-102 were plated with autoautologous APC MNC, as above, in the presence of monoclonal antibodies (mAbs) (final concentration of 1:100) recognizing different MHC Class II gene products. (The nomenclature used for the antibodies is from the Tenth International Histocompatibility Workshop; their specificity is also given). The results are set forth in Table 10 below.

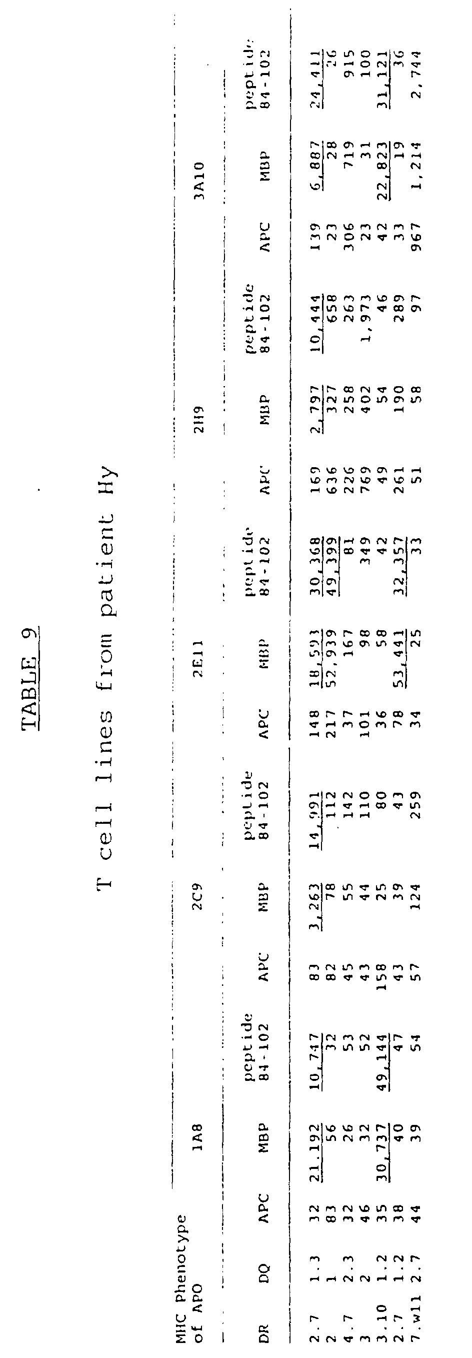

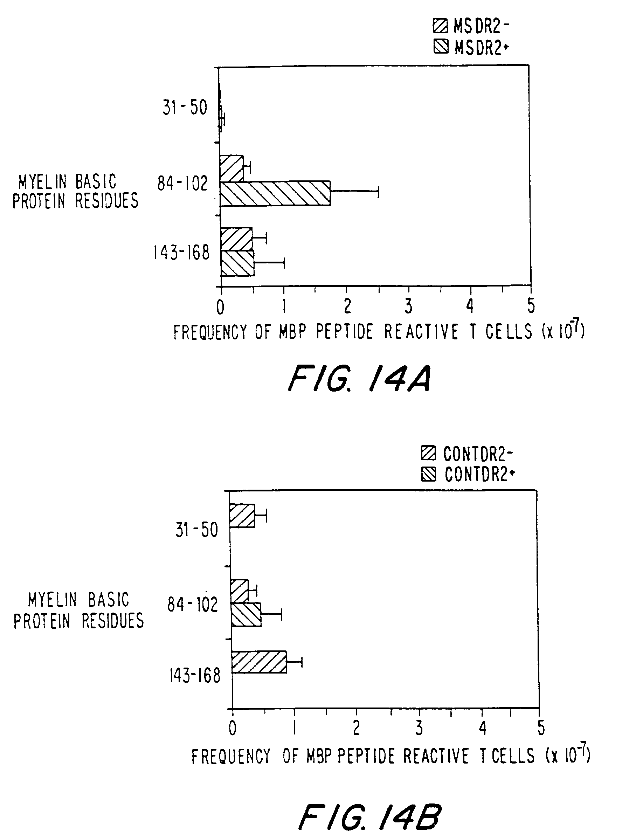

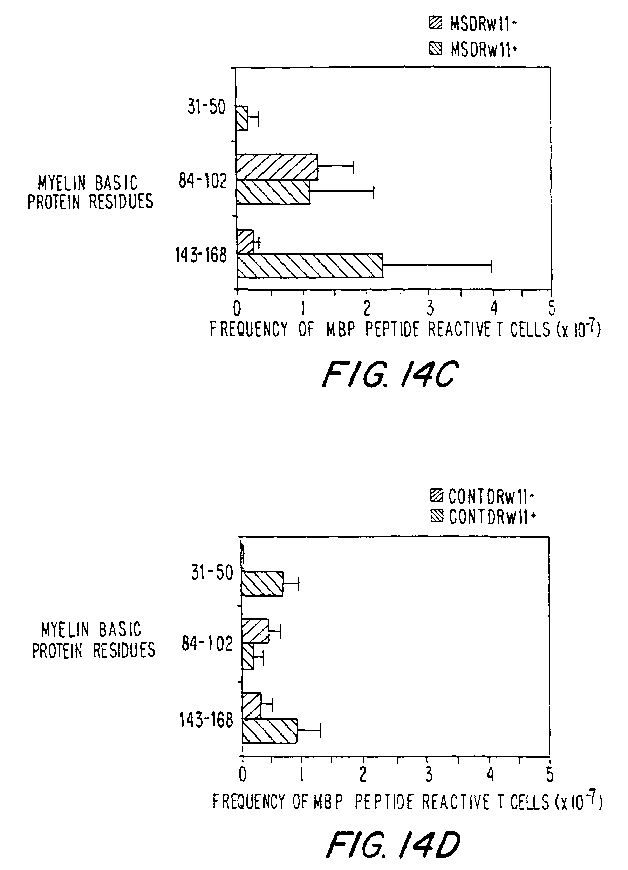

The frequency of peptide specific cell lines from normal subjects and other neurologic disease controls were virtually identical and thus combined for analysis. The mean frequency of T-cell lines from subjects with MS that were selectively reactive to MBP residues 84-102 was higher as compared with controls (Figure 1). Significant but less striking increases in reactivity to MBP residues 61-82 and 124-142 were also observed in MS patients, while both MS and control subjects showed high frequencies of T-cell lines reactive with MBP residues 143-168. The DR2, DQw1 haplotype was very infrequent in the control subjects and more common in patients with MS (Table 9). An association was observed between the DR2 phenotype and both the proportion or the frequency of T-cell lines reactive to MBP residues 84-102 (Figure 14).

To determine if T-cell reactivity to MBP residues 84-102 was associated with DR2, DQw1 expression in non-MS subjects, an additional 6 normal subjects with DR2, DQw1 phenotype were investigated. The results are shown in Figure 14.

A DR2 association was also observed among controls in terms of the proportion of T-cell lines reactive with MBP residues 84-102 (DR2+ controls, 31.0±10-.8%; DR2-, 10.1±0.4%), though the total frequency of lines reactive with this region of MBP was less than that in patients with MS (Figure 14). Though DQw1 is in linkage dissociation with DR2 as well as with DR1 and DRw10, independent analysis of peptide reactivity revealed no association with DQw1 phenotype expression.

The DRw11 phenotype was more common in controls than in subjects with MS (Table 8A). DRw11 was positively associated with the frequency of lines reactive to MBP residues 142-168 in patients with MS and controls, but not with the frequency of lines reactive with MBP residues 84-102 (Figure 13). Reactivity to MBP residues 31-50, which was predominantly observed in control subjects, was associated with DRw11. Other MHC associations were not observed.

The MHC association with residues of the T-cell lines reactive with an immunodominant MBP epitope was determined. The results are set forth in Table 10 below. More specifically, it was determined whether the MHC haplotypes were used to present antigen in the T-cell lines reactive with an immunodominant MBP epitope.

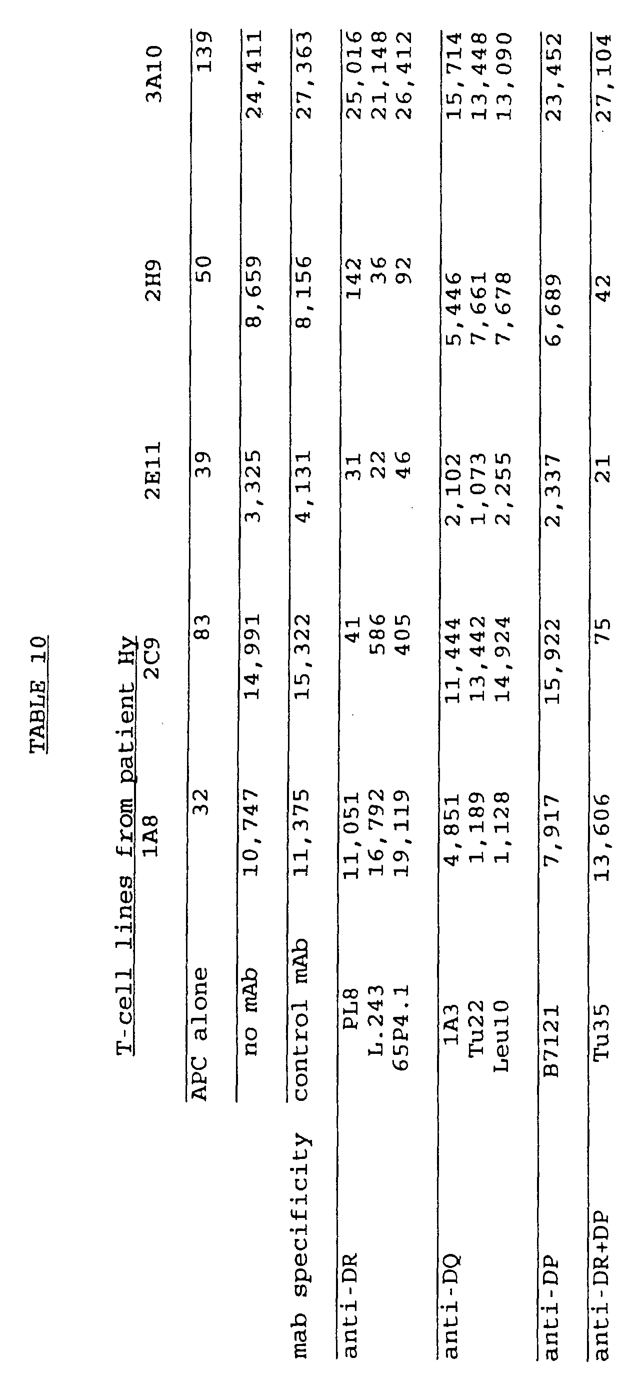

Monoclonal antibody blocking studies of five T-cell lines reactive with MBP residues 84-102 suggested that both DR and DQ molecules could function as restricting elements. Among clones blocked by anti-DR mAb, clone 2E11 proliferated in response to MBP residues 84-102 with the panel of DR2+ APC while 2C9 and 2H9 proliferated only with autologous APC (Table 10). The recognition of peptide by clones 1A8 and 3A10, which were partially blocked by anti-DQ mAbs was restricted to APC from the responder and one of two APC donor subjects expressing DQw1.

To investigate further the relationship between MHC expression and frequency of T-cell reactivity to immunodominant MBP epitopes, a family with one afflicted sibling expressing both DR2 and DRw11 phenotypes was studied.

The family members of an MS patient expressing the DR2, DQw1; DRw11, DRw52, DQw1 Class II MHC haplotypes were examined for the frequency of T-cell lines reactive with MBP residues 84-102 and 143-168.

A total of 1,728 individual T-cell lines were generated from both parents and 4 siblings and the number of lines reactive with either MBP peptide 84-102 or 143-168 were determined.

2x10⁵ MNC in each of 288 wells (three 96 well round bottom plates) were cultured with MBP (10 micrograms/ml) as outlined above for each subject. On day 16, each T-cell line was analyzed for reactivity to synthetic peptides corresponding to the MBP residues 84-102 and 143-168. The number of lines reactive with each peptide (stimulation index SI>3, delta CPM>500) generated per subject are shown. The actual stimulation indices were generally >20. P1 and P2=parents; S1-S3=siblings. The results are set forth in Table 11 below.

**TABLE 11**

| | PATIENT | P1 | P2 | S1 | S2 | S3 |
|---|---|---|---|---|---|---|
| | | DR4 | | DR4 | DR4 | |
| | DR2 | DRw53 | DR2 | DRw53 | DRw53 | DR2 |
| | DQw1 | DQw3 | DQw1 | DQw3 | DQw3 | DQw1 |
| | DRw11 | DRw11 | DRw6 | DRw6 | DRw6 | DRw4 |
| | DRw52 | DRw52 | DRw52 | DRw52 | DRw52 | DRw53 |
| | DQw1 | DQw1 | DQw1 | DQw1 | DQw1 | DQw3 |

| MBP peptide | | | | | | |
|---|---|---|---|---|---|---|
| 84-102 | 49 | 1 | 4 | 6 | 1 | 7 |
| 143-168 | 41 | 14 | 0 | 3 | 2 | 2 |

The DR2+, DRw11+ patient had a high frequency of T-cell lines reactive to both MBP residues 84-102 and 143-168. The DRw11+ parent preferentially recognized MBP residues 143-168, while the DR2+ parent preferentially recognized MBP residues 84-102. The frequency of MBP peptide reactive lines, however, was lower than that of the patient. One sibling was DR2+ and preferentially recognized MBP residues 84-102. Of two HLA identical siblings with DR4, DQw3/DRw6, DQw1, one reacted to MBP peptide 84-102 whereas the other did not. Although DQw1 may restrict recognition of MBP residues 84-102, other factors such as inherited TCR polymorphism may have influenced T-cell reactivity to the MBP autoantigen in one of the DR4, DQw3/DRw6, DQw1 siblings. This family linkage analysis suggested that optimum recognition of immunodominant MBP epitopes requires specific Class II MHC alleles both in patients with MS and in controls. In total, these studies indicate that although control subjects expressing DR2 appear to preferentially recognize the same MBP determinant as compared to DR2+ MS patients, their frequency in the blood is less than that of patients with MS.

### EXAMPLE 7A: SEQUENCING OF VB17 TCR

The T-cell receptor VB17+ PCR products from six cloned T-cell lines were sequenced by the dideoxy method as described reactive with MBP residues 84-102 (Patients Hy, Fr and Ns) in Example 5. The DNAs were amplified using PCR primers for the VB17-leader sequence and the TCR CB region described above in Example 5. The amplified DNA was cloned into M13 and sequenced using the well-known dideoxy method (3 M13 plaques per T-cell line). The results are set forth in Table 12 below.

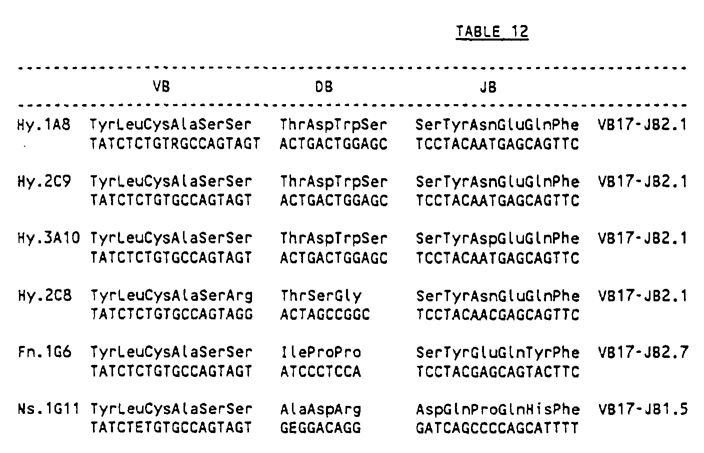

It should be noted above that the VB17 sequence of all 4 T-cell lines established from MS patient Hy were 100% homologous to the published VB17 sequence.

The following is a concordance between the 3-letter and the 1-letter codes for aminoacids. It is provided for convenience.
Aspartic acid
   (Asp, D)
Glutamic acid
   (Glu, E)
Lysine
   (Lys, K)
Arginine
   (Arg, R)
Histidine
   (His, H)
Tyrosine
   (Tyr, Y)
Cysteine
   (Cys, C)
Asparagine
   (Asn, N)
Glutamine
   (Gln, Q)
Serine
   (Ser, S)
Threonine
   (Thr, T)
Glycine
   (Gly, G)
Alanine
   (Ala, A)
Valine
   (Val, V)
Leucine
   (Leu, L)
Isoleucine
   (Ile, I)
Methionine
   (Met, M)
Proline
   (Pro, P)
Phenylalanine
   (Phe, F)
Tryptophan
   (Trp, W)

Example 8: In the Example presented below, the following materials and methods were used:

Generation of MBP readtive T-cell clones. Myelin basic protein (MBP) reactive T-cell lines and clones were generated as described previously (5). T-cell clones were generated from MBP 84-102 reactive T-cell lines by limiting dilution (0.3 cells/well) in 96 well v-bottomed microtiter plates (Costar, Cambridge, MA) in the presence of µg/ml PHA.P (Wellcome Diagnostics, Beckenham, UK) and 10⁵ irradiated allogenetic PBMC in media consisting of RPMI 1640 (Whittaker, Walkersville, MD), 10% pooled human AB serum (PHS) (Biocell, Carson City, CA), 4mM glutamine (GIBCO, Grand Island, NY), 10 mM HEPES (Whittaker), 100µ/ml penicillin/streptomycin (GIBCO), 5% IL-2 (Human T stim, Collaborative Reseach, Bedford, MA), and 1 µ/ml rIL-4 (kindly supplied by Genetics Institute, Cambridge, MA). Clones were restimulated every 8-12 days at 10⁴/well in 96 well round-bottomed microtiter plates (Costar) with alternating rounds of 40 µM 84-102 pulsed, irradiated autologous PBMC and PHA/allogeneic PBMC. For the experiments shown here, T-cell clones were restimulated from aliquots of the same freeze to prevent drift from long term culture. T-cells were frozen in 90% FCS/.10% DMSO in liquid nitrogen.

MBP PEPTIDE 84-102. MBP PEPTIDE 84-102, amino acid sequence DENPVVHFFKNIVTPRTPP was systhesized by the solid phase method (Biosearch Lab, Inc., San Raphael, CA) and purified on HPLC as previously reported (5).

Cell lines. Previously established EBV transformed B cell lines 9010 (DR2w2, DQI) and 9009 (DR2w21, DQ1) were grown in 10% FCS complete media then frozen as described above and thawed immediately prior to antigen pulse. Mouse fibroblast L-cell line transfected with DR2a (Idnd gift of R. Sekaly) were grown in DMEM (Whittaker), 10% FCS (Whittaker) 1.6 mM xanthine (Sigma Biochemicals, St. Louis, MO), 110 µM hypoxanthine (Sigma), 18 µM mycophenolic acid (GIBCO) selection media, frozen as described above, and thawed immediately prior to antigen pulse. HT-2 murine IL-2 dependent T-cell line (ATTC) was grown in 10% FCS complete media with 5% IL-2 and used for IL-2 bioassay two days following last feeding.

MAbs. MAbs used in these studies include CD3 specific OKT3 and 2AD2, CD2 specific T11₂ and T11₃ (kind gifts from S. Schlossman), HLA-DR specific L243, HLADQ specific S3/4 (kind gifts from F. Bach), HLA-DP specific B7/21 (kind gift from N. Flomenberg), CD28 inactive 9.3 (kind gift from J. Ledbetter), CD45RA inactive 2H4 (Schlossman), CD45RO specific UCHL-1 (Dakopatts, Glostrup, Denmark) LFA-3 and LFA-1 (kind gifts from T. Springer), and CD25 Tac (kind gift of T. Waldman).

Proliferation assay. T-cell clone (10⁵/well) was plated in triplicate and cocultured with appropriate stimuli desipated in figure legends for 72 hours at 37° C, 90% humidity, 5% CO₂, in 96 well flat bottomed microtiter plates (Costar) pulsed with 2 µCi [³H]TdR (2 Ci/mmole, New England Nuclear Boston, MA) for the last 18 hours of culture. APCs were prepared by pulsing B cells or L-cells at 10⁶ cells/ml in complete media either in the presence or absence of 40 µM MBP 84-102 for 2 hours at 37° C, washing twice with 4° C Hanks (Whittaker), followed by irradiation with 5000 rad. at 4° C. To stimulate T-cells without acessory APCs, 2 µM MBP peptide 84-102 or 10³ µ/ml rIL-2 (Hoffman LaRoche, Nutley, NJ) was added directly to the cells for the duration of the culture.

Flow cytometric analysis of T-cells. A 1/100 dilution of the MAb ascites in PBS/2% PHS was used to coat T-cells at 10⁶/ml for 30 min. at 4°C. Cells were washed twice with 4° C staining media, the stained with 1/60 FITC conjugated goat anti-mouse Ig (Tago, Burlinganie, CA) for 30 min. at 4° C. Cells were washed twice as above, then fixed with 1% formaldehyde (J.T. Baker Chemical Co, Phillipsburg, NJ). Flow cytometric analysis was performed on an Epics C flow cytometer (Coulter Electronics, Hialeah, FL).

B7-Transfection. 50 µg KpnI linearized B7-pCDM8 construct was cotransfected with 5 µg of PvuI linearized POP.F into L-tk⁻ cells or into DR2⁺ L-tk cells (previously transfected with DR2) by electroporation using the BRL electroporator at settings of 250 V and 1600 mF. The POP.F plasmid contains the herpes simplex virus thymidine kinase gene under the control of the SV40 promoter (23). DR2⁻B7⁺ transfectants were selected by growth in hypoxanthine-aminopterin-thymidine (Sigma) containing media and cloned. DR2⁺B7⁺ transfectants were selected and cloned in xanthine/hypoxanthine/mycophenolic acid media containing 150 µg/ml G418 sulfate (GIBCO). Clones expressing cell surface B7, as assayed by indirect immunofluorescence with anti-B7 mAb, were recloned.

Measurement of [Ca+2]i. As described previously (22), T-cell clones (0.2-1.0 x 10⁷/ml) were loaded with 2 µg/ml Indo-1 (Sigma) in culture media for 45 min. at 37° C. Indo-loaded cells were diluted 1/10 with media and kept at 4° C until 1 min prior to FACS analysis. Stimulator APCs were pulsed with or without 40 µM 84-102, washed twice and resuspended in media at 4° C. Unstimulated Indo-loaded T-cells were analyzed for 30 seconds prior to addition of stimulus. In the case of cellular stimulators, stimulator APCs + loaded responders were analyzed for 30 seconds, then centrifuged for 1 minute at 1500 RPM to establish cell-cell contact, then resuspended and analyzed for response. Ionomycin (100 µg/ml) (Sigma) used as a positive control for Indo-1 loading.

Detection of cytokine mRNA by northern analysis. T-cell clone Ob.1A12.8 (10⁵/well in 96-well round-bottomed microtitier plates in Il-2/IL-4 supplemented media) were grown in the presence or absence of 2 µM 84-102 for 48 hours. Cells were washed, and resuspended in complete media at 2 x 10⁶/ml in the presence or absence of either 10 ng/ml PMA and 1µg/ml ionomycin, 2 µM 84-102, or 2 µm 84-102 + 10 ng/ml PMA for 4 hours at 37°C. Total cellular RNA was extracted using the RNAzol B method (TM Cinna Scientific, Friendswood, TX). 10 µg of total cellular RNA was fractionated by formaldehyde gel electophoresis using 1.2% SeaKem ME agarose (FMC Bioproducts, Rockland, ME) and 2.2 M formaldehyde. After fractionation, RNA was blotted onto Nytran membranes (Schleicher & Schuell, Keene, NH) with a 10X SSC solution by capillary transfer overnight. Membranes were baked in a vacuum oven at 80° C for 2 hrs. For hybridization of cytokine probes, membranes were prewashed in 0.5x SSC and 5%SDS at 65° C for 2 hr, then prehybridized in 50% formaldehyde, 5xSSC, 0.5% SDS, 1x Denhardts' solution, 10% dextran sulfate, and 100 µg/ml salmon sperm DNA (Sigma) at 42° C for 1-2 hrs. Probes for IL-2, IL-4, and γIfn (described previously in ref. 24) were labelled to a specific activity of >10⁸ cpm/µg by random primer labelling method (Boehringer Mannheim, Mannheim, W. Germany) and added to fresh hybridization buffer. Hybridizations were performed at 42° C for 20 hrs. Filters were washed in 0.2x SSC/O.1% SDS twice for 10 min. at 24° C, then twice for 30 min. at 50° C. Autoradiography was performed at -70° C for 1-5 days.

### Results

T-cells previously stimulated with peptide antigen are unresponsive to antigen. We have previously shown that MBP reactive T-cell clones proliferate in response to peptide antigen in the absence of traditional APCs by presenting peptide antigen on their own MHC class II molecules to autologous T-cell clones (22). We then examined whether T-cell presentation of antigen involved different signalling events in responding T-cell clones as compared to traditional APCs. As shown in Figure 15, T-cell clone Ob.1A12.8 responded as well to MBP peptide 84-102 added directly to the culture as compared to peptide bound to pulsed DR2⁺ B cell line or DR2 transfected L-cells in a primary proliferation assay (Figure 15A). However, T-cells originally stimulated by free MBP 84-102 peptide antigen were unresponsive to antigen stimulation in any form when assayed one week later (Fig. 15B, open circles), while T-cells stimulated in primary cultures by peptide pulsed B cells or DR2⁺ L-cells responded normally to secondary stimulation (Figure 15B, closed symbols). The degree of unresponsiveness in the secondary stimulation was inversely proportional to the antigen concentration and the proliferation induced in the primary stimulation.

We have found MBP peptide induced unresponsiveness in 5 different T-cell clones from two different individuals reactive against two different MBP peptide epitopes. Only the specific peptide epitope induces T-cell unresponsiveness (data not shown).

The addition of costimulatory cells does not reverse unresponsiveness. We hypothesized that the induction of anergy was due to a negative signal rather than the lack of a costimulatory signal on T-cell APCs since L-cells (which presumably have no human costimulator molecules) did not induce anergy. However, L-cells have been recently found to express murine B7 (G. Freeman, manuscript in preparation) and murine B7 has been shown to costimulate human T-cells (25). To directly test the costimulatory effect of L-cells on the induction of unreponsiveness, we added DR2 transfected L-cells to the culture of the T-cell clone with unbound peptide antigen. To directly test the role of human B7 as a costimulator molecule, the human B7 gene was transfected into DR2⁺ and Dr2⁻ L-cells. As shown in Table 5, T-cells cultured for a week in 84-102 were not responsive to secondary antigenic stimulation even when L-cells expressing human B7 or coexpressing B7 and DR2 were added to the culture.

To test if the unresponsiveness induced by peptide antigen was reversible by costimulator molecules other than B7, we added irradiated B7⁺B cells that were either the appropriate (9010) or inappropriate (9009) HLA-DR for antigen presentation to the culture of T-cell clone with MBP 84-102. Figure 16 demonstrates that neither B-cell line was able to prevent the induction of anergy caused by T-cells responding to free peptide. However, as shown in Figure 15, stimulation with MHC matched B-cell line 9010 that had been pulsed with peptide antigen and washed prior to culture did not lead to the unresponsiveness of the T-cell clone. Alothough the T-cell clone lost antigen responsiveness following exposure to peptide antigen, the response to IL-2 was unchanged, indicating that the unresponsiveness was not due to cell death.

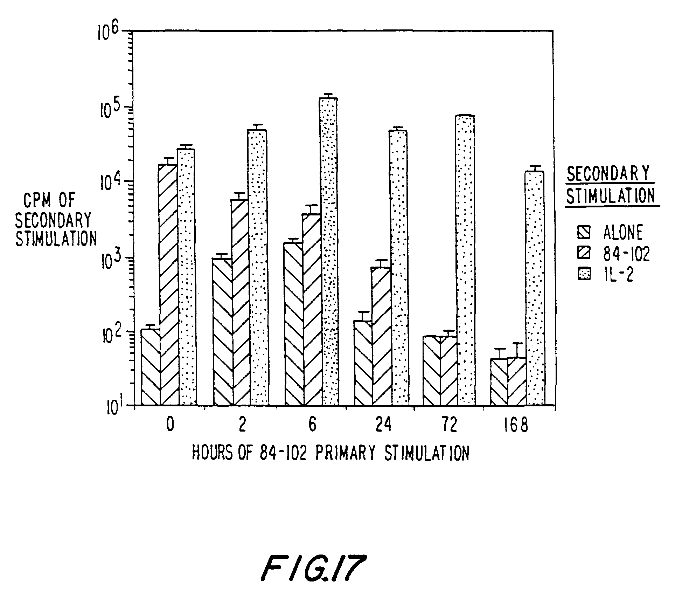

Kinetics of anergy induction. To determine the kinetics of anergy induction, secondary T-cell response to MBP 84-102 peptide and IL-2 was assayed following a primary stimulation with peptide from 2 to 168 hours. A greater than ten fold reduction in the response to antigen was induced within 24 hours of MBP 84-102 peptide culture, by which time the background proliferation (alone) had returned to that of resting cells. This unresponsiveness was even further enhanced to >100 fold reduction of the response over 4 days and was maintained for the duration of the experiment, 7 days (Figure 17).

Unresponsive T-cells continue to express CD3. The unresponsiveness of peptide anergized T-cell clones could have been secondary to a loss of CD3/TcR cell surface expression. This was not the case, as T-cells rendered unresponsive by culture with peptide antigen had an equivalent CD3 surface expression as compared with non-anergized T-cell clones (Figure 8).

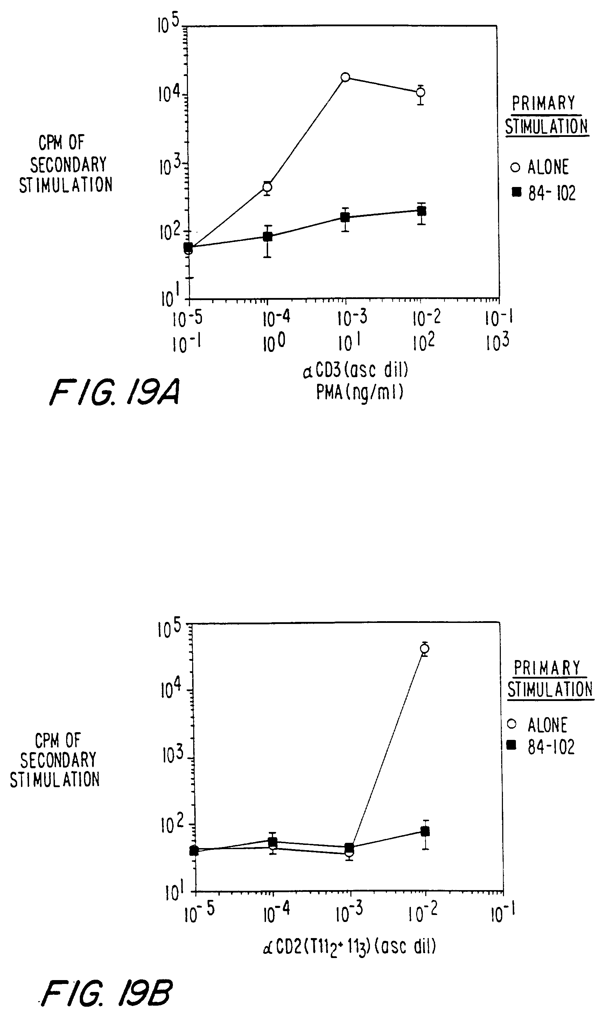

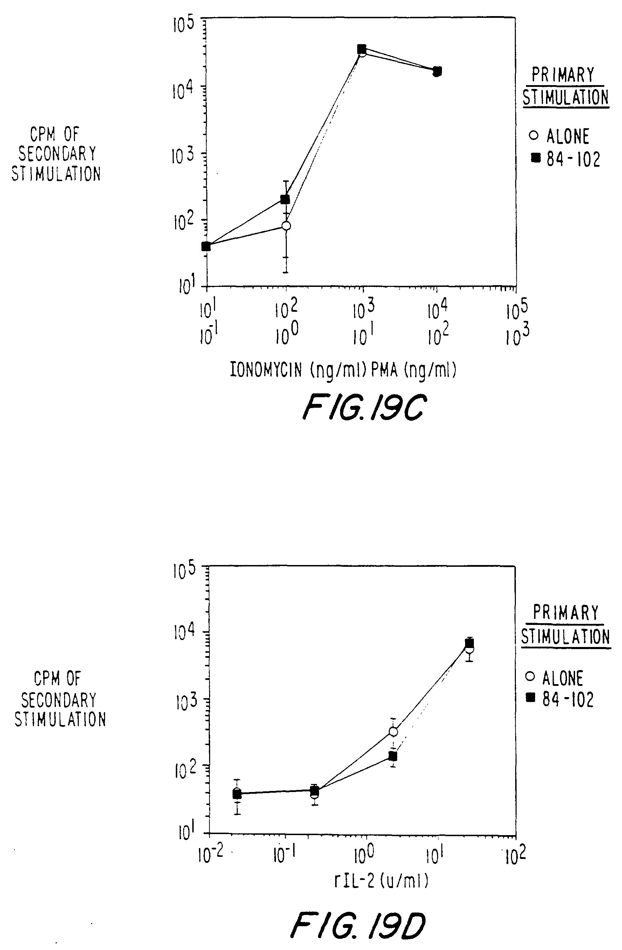

Response of anergized T-cell clone to nonantigenic stimuli. To more precisely define which activation pathways are defective in anergized T-cell clones, T-cells anergized by peptide culture were treated with reagents which either mimic or bypass cell surface antigenic stimulation (Figure 19). Anergized T-cells failed to proliferate in response to either the combination of αCD3 and PMA or the CD2 mitogenic MAbs T11₂ and T11₃. In contrast, anergized T-cells responded normally to the combination of PMA and ionomycin, which bypasses the need for transmembrane signalling (26), and to rIL-2 which stimulates proliferation through a separate pathway (27).

Anergized T-cells are defective in their ability to release calcium following antigenic stimulation. As we have previously shown that MBP reactive T-cell clones release intracellular [CA⁺²]i in response to peptide presented by antigen pulsed T-cells or B cells (22), we tested the ability of peptide anergized T-cells to respond in this assay. Anergized T-cells have a marked reduction in their release of intracellular [Ca⁺²]i in response to either TCR crosslinking with αCD3 or peptide antigen presented by B cells (Figure 20). In contrast, the response to ionomycin in anergized T-cell clones is equivalent to that of the non-anergized T-cell clone.

Anergized T-cells fail to produce cytokines in response to antigen. T-cell clones either cultured alone or anergized with MBP peptide 84-102 for 48 hours in a primary culture were stimulated in a secondary culture with either peptide, peptide + PMA, or PMA + ionomycin. mRNA was extracted after 4 hours and the relative quantity of IL-2, IL-4, and γIFN examined by northern blotting. Prior to the induction of anergy, T-cell clone Ob.1A12.8 synthesized IL-2, IL-4, and γIFN mRNA in response to MBP 84-102 peptide or to a combination of PMA + ionomycin or peptide + PMA. In contrast, the anergized T-cell clone failed to synthesize IL-2, IL-4, and γIFN mRNA in response the MBP 84-102 peptide alone or to the peptide in combination with PMA, while synthesizing significant cytokine mRNA in response to PMA + ionomycin stimulation (Figure 21A). The results of the northern analysis of IL-2 mRNA were confirmed by an IL-2 bioassay using HT-2 cells (Figure 21B). Anergized T-cells activated with peptide antigen in a secondary stimulation did not secrete IL-2, whereas the non-anergized did secrete IL-2, as measured by the proliferation of HT-2 cells.

Traditional APCs such as macrophages, B cells, and dendritic cells constitutively express MHC class II and are able to process and present protein antigen to CD4+ T-cells (28). Human T-cells which express MHC class II following activation are able to present peptide or degraded antigen but normally process whole antigen suggesting that they act as "nontraditional" APCs (22,29-31). Although T-cell presentation of MBP 84-102 results in a proliferation assay, here we show that T-cells stimulated with peptide antigen are unresponsive to secondary stimulation by either antigen or TcR/CD3 crosslinking, but no IL-2. Moreover, long lasting antigen unresponsiveness was induced with unbound peptide but not peptide pulsed B cells or DR2+L-cell transfectants, suggesting that the unresponsiveness was not merely due to an unresponsive refractory period following prior stimulation as has been suggested as a mechanism of high dose tolerance (9). We refer to this unresponsiveness as "anergy" because the T-cells are unresponsive to antigenic stimulation while remaining IL-2 responsive, which were the original criterion established to define the term (8).

T-cell tolerance can be induced in vitro by chemically fixed APC or immobilized αCD3 mAb where a signal is delivered through the TcR/CD3 complex without a second essential costimulator signal (6-8). It has been shown that the CD28 molecule on T-cells,interacting with B7 on B cells and activated macrophages is a component of the costimulatory pathway necessary for T-cell activation and postulated that the absence of B7 costimulation may lead to anergy (14-16). However, T-cell anergy induced by self T-cell presentation of MBP peptide appears to occur in the presence of costimulation because the addition of either B7 transfectants or EBV transformed B cells, which express high levels of B7, were unable to prevent the induction of unresponsiveness. Moreover B cells which have the appropriate MHC and act and nontoleragenic APCs in the absence of free antigen and presumably compete for presentation of peptide antigen with T-cells during culture, are not able to overcome the induction of unresponsiveness. These results suggest that the induction of anergy by T-cell presentation of peptide antigen results in a negative signal rather than the lack of a positive costimulation. These results also have practical implications for the growth of antigen specific T-cell lines and clones which should be restimulated in the absence of free peptide antigen to prevent the loss of antigen responsiveness.

The mechanism of anergy induced by T-cell presentation of autoantigen was examined. Anergized T-cells have a marked dimunition in their ability to release [Ca+²]ᵢ in response to either APCs or αCD3 mAb crosslinking. In addition, treatment with the combination of PMA and ionomycin completely restored the cytokine production and proliferation of anergized T-cells, suggesting that the signalling events following PKC activation and [Ca+²]ᵢ release are normal. Thus, the state of anergy as defined by alterations in signal transduction in our system are different from studies on in vitro clonal anergy by Jenkins and Schwartz where no signalling defects were observed in membrane proximal events (32,33). The mechanism of T-cell anergy induced by T-cell presentation of antigen appears similar to studies of transgenic mice in which autoreactive T-cells that escape into the periphery fail to release [Ca+²]ᵢ in response to TcR signalling but are able to proliferate in response to the combination of PMA and ionomycin (21). Therefore, there appear to be at least two different states of functionally defined anergy. Just as cell transformation can occur by the alteration of one of many different molecules which regulate a cell's signal transduction pathway, the phenomenon of T-cell anergy may be acheived by several different mechanisms which affect different stages of T-cell activation.

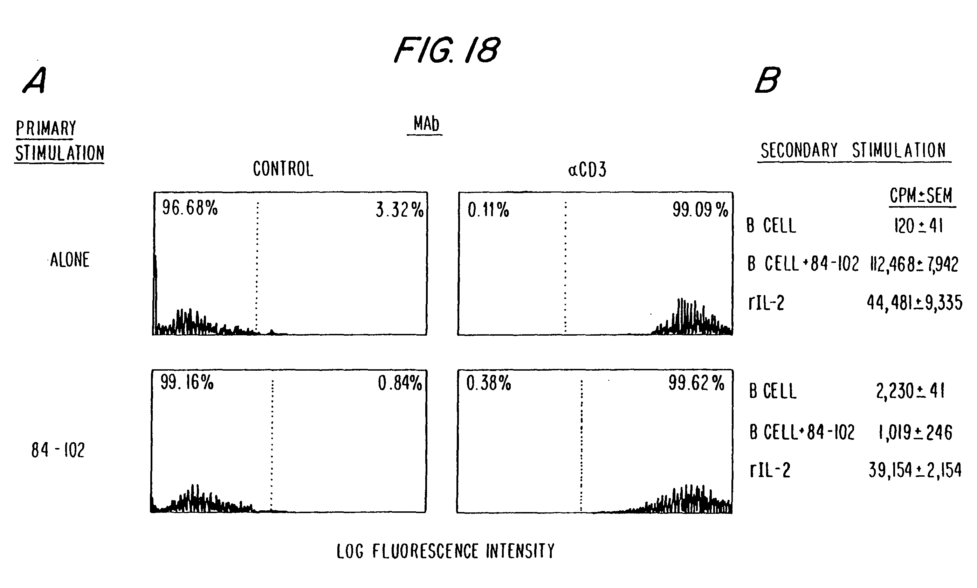

O'Hehir and coworkers have shown that T-cell unresponsiveness induced by peptide or superantigen can be characterized by a decrease in the surface expression of CD3 together with an increase in CD25 16 hours after the addition of peptide antigen (34,35). We also observe a moderate decrease in CD3/TCR expression and increase in CD25 expression at this early time point, presumably due to T-cell activation (data not shown). However, we find that by 4 days anergized T-cells have equivalent cell surface density of CD3 as non-anergized T-cells (Figure 18). This indicates that anergy cannot be explained simply as the reduction of cell surface CD3/TCR.

As we have seen with the majority of human T-cell clones derived from peripheral blood (24), T-cell clone Ob. 1A12.8 appears to be a T_{H}O phenotype in its ability to produce cytokines of both the T_{H}1 and T_{H}2 subsets in response to mitogen stimulation (36). Anergized T-cells were unable to synthesize IL-2, IL-4, or γIFN mRNA or secrete measurable IL-2 in response to antigenic stimulation wither with or without PMA, while the addition of both ionomycin and PMA generated cytokine synthesis. These results are consistant with both proliferation and CA+² flux data suggesting that the block in T-cell signalling is in an event preceding Ca+² mobilization. The negative signal generated by T-cells in the response to peptide antigen is yet to be defined, as is the actual biochemical signalling event which is altered in these anergized T-cells.

A major question in immunology relates to how activated autoantigen, reactive T-cells are regulated during an inflammatory response. In previous work, we have shown that T-cells are able to present peptide antigen and partially degraded native MBP, although they are unable to process and present highly purified MBP (22). This led us to speculate that activated T-cells in an inflammatory site (such as a demyelinating plaque in the brain) may be able to present fragments of MBP to other T-cells at the site. The demonstration that T-cell presentation of peptide antigen lead to clonal unresponsiveness suggests that this mechanism of tolerance may have evolved to prevent autoreactive T-cells in an inflammatory site from clonally expanding to damaged self tissue. A high extracellular concentration of degraded protein would be characteristic of self antigen, while foreign antigen after the initial in situ immune response would be present at a lower concentration and become internalized into traditional APCs for processing and presentation. In this way, inappropriate antigen presentation by T-cells which can present but not process the antigen would lead to anergy instead of activation of the responding T-cell clone in an inflammatory site where degraded self antigen is available.

In conclusion, we present evidence for a mechanism to explain the initial observation of Lamb and Feldman (10,11) that pretreatment of T-cell clones with peptide antigen leads to antigen unresponsiveness. Anergy is related to the expression of MHC class II by activated human T-cells which are able to present peptide antigen to autologous T-cells. Although this interaction leads to a primary stimulation of the T-cells, they are rendered unresponsive to subsequent stimulation within 24 hours of peptide pretreatment. The signalling defect in anergized T-cells is membrane proximal because peptide treated T-cell clones are inhibited in their ability to release [Ca+²]ᵢ, produce cytokines, and proliferate to antigen stimuli, but exhibit a normal response to treatment with PMA and ionomycin.

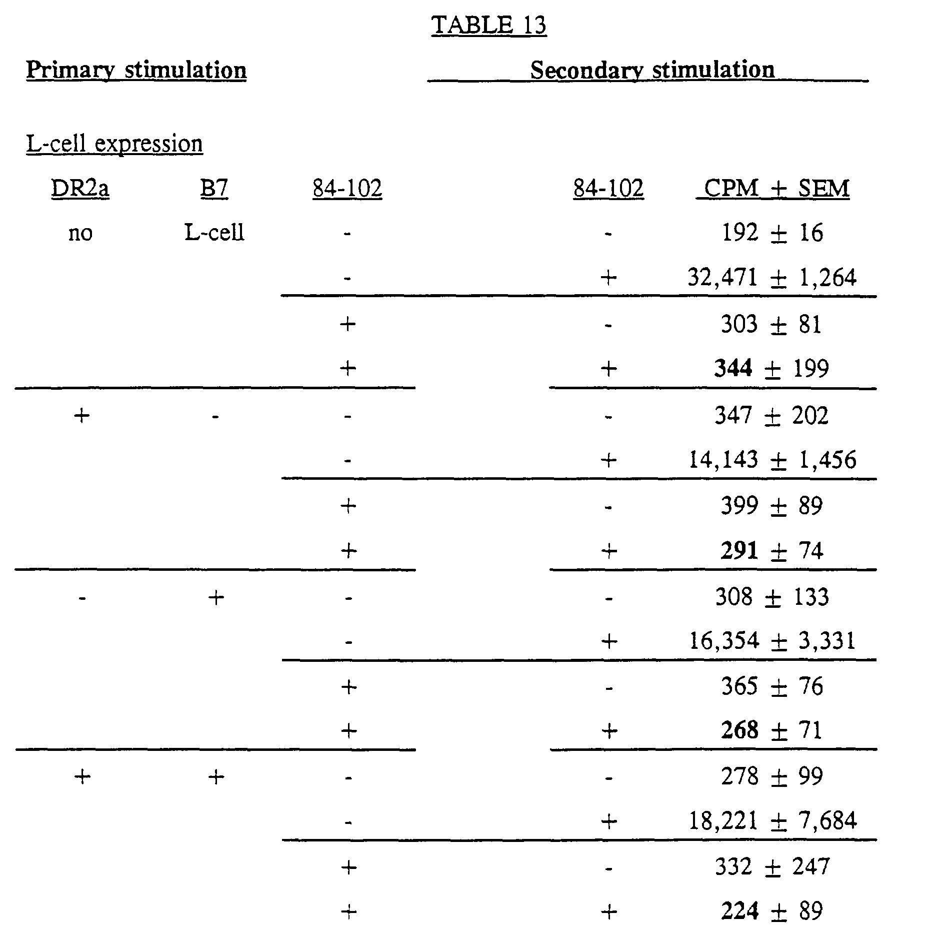

Table 13. Anergy occurs in the presence of B7 accessory cells. T-cell clone Ob.2F3. was cultured for 7 days in the presence or absence of 5 µg/ml 84-102 with or without the additio of 10⁴/well L-cells (irradiated at 5000 rad) with expression of DR2 and/or B7 transfected molecules. L-cell lines with and without expression of DR2 were transfected with B7. Mean fluorescence intensity was 77.2 (mouse Ig-PE) and 215.6 (B7-PE) for the DR2⁺B7⁺ line and 56.8 (mouse Ig-PE) and 158.6 (B7-PE) for the DR2⁻B7⁺ line. Following the primary culture, T-cells were washed and assayed for proliferation to 84-102.

### List of references belonging to the specification in Example 8

1. Kappler, J.W., U.D.Staerz, J. White, and P.C. Marrack. 1988. Self-tolerance eliminates T-cells specific for Mls-modified products of the major histocompatibility complex. Nature, 332:35.
2. MacDonald, H.R., R. Schneider, R.K. Lees, R.C. Howe, H. Acha-Orbea, H. Festenstein, R.M. Zinkernagel, and H. Hengartner. 1988. T-cell receptor Vβ use predicts reactivity and tolerance to Mlsa-encoded antigens. Nature, 332:40.
3. Kappler, J.W., N. Roehm, and P. Marrack. 1987. T-cell tolerance by clonal elimination in the thymus. Cell, 49:273.
4. Kiesielow, P., H. Bluthman, U.D. Staerz, M. Steinmetz, and H. von Boehmer. 1988. Tolerance in T-cell receptor transgenic mice involves deletion of nonmature CD4+8+ thymocytes. Nature, 333:742.
5. Ota, K., M. Matsui, E.L. Milford, G.A. Macklin, H.L. Weiner, and D.A. Hafler. 1990. T-cell recognition of an immunodominant myelin basic protein epitope in multiple sclerosis. Nature, 346:183.
6. Jenkins, M.K., J.D. Ashwell, and R.H. Schwartz. 1988. Allogeneic non-T spleen cells restore the responsiveness of normal T-cell clones stimulated with antigen and chemically modified antigen presenting cells. J. Immunol. 140:3324.
7. Meuller, D.L., M.K Jenkins, and R.H. Schwartz. 1989. Clonal expansion versus clonal inactivation: A costimulatory signalling pathway determines the outcome of T-cell antigen receptor occupancy. Ann. Rev. Immunol. 7:445.
8. Jenkins, M.K., D.M. Pardoll, J. Mizuguchi, H. Quill, and R.H. Schwartz. 1987. T-cell unresponsiveness in vivo and in vitro: Fine specificity of induction and molecular characterization of the unresponsive state. Immunol. Rev. 95:113.
9. Suzuki, G., Y. Kawase, S. Koyasu, I. Yahara, Y. Kobayashi, and R.H. Schwartz. 1988. Antigen-induced suppression of the proliferative response of T-cell clones. J. Immunol. 140:1359.
10. Lamb, J.R., B.J. Skidmore, N. Green, J.M. Chiller, and M. Feldmann. 1983. Induction of tolerance in influenza virus-immune T-lymphocyte clones with synthetic peptides of influenza hemagglutinin. J. Exp. Med. 157:1434.
11. Lamb, J.R., and M. Feldmann. 1984. Essential requirement for major histocompadability complex recognition in T-cell tolerance induction. Nature 308:72.
12. Kennedy, M.K., M.C. Dal Canto, J.L. Trotter, and SJ. Miller. 1988. J. Immunol. 141:2986.
13. Williams, I.F., and E.R. Unanue. 1990. Costimulatory requirements of murine Th1 clones. The role of accessory cell-derived signals in response to anti-CD3 antibody. J. Immunol. 145:85.
14. Gimmi, C.D., G.J. Freeman, J.G. Gribben, K. Sujgita, A.S. Freedman, C. Morimoto, and L.M. Nadler. 1991. B-cell surface antigen B7 provides a costimulatory signal that induces T-cells to proliferate and secrete interleukin 2. Proc. Natl. Acad. Sci. USA. 88:6575.
15. Linsley, P.S., W. Brady, L. Grosmaire, A. Aruffa, N.K. Damle, and J.A. Ledbetter. 1991. Binding of the B-cell activation antigen B7 to CD28 costimulates T-cell proliferation and interleukin 2 mRNA accumulation. J. Exp. Med. 173:721.
16. Freedman, A.S., G. Freeman, J.C Horowitz, J. Daley, and L.M. Nadler. 1987. B7, a B-cell restricted antigen that identifies preactivated B-cells. J. Immunol. 139:3260.
17. Burkly, L.C., D. Lo, O. Kanagawa, R.L. Brinster, and R.A. Flavell, 1989. T-cell tolerance by clonal energy in transgenic mice with nonlymphoid expression of MHC class II I-E. Nature, 342:564.
18. Lo, D., L.C. Burkly, G. Widera, C. Cowing, R.A. Flavell, R.D. Palmiter, and R.L. Brinster. 1988. Diabetes and tolerance in transgenic mice expressing class II MHC molecules in pancreatic beta cells. Cell, 53:159.
19. Webb, S., C. Morris, and J. Sprent. 1990. Extrathymic tolerance of mature T-cells: clonal elimination as a consequence of immunity. Cell. 63:1249.
20. Ramsdell, F., T. Lantz, and B.J. Fowlkes. 1989. A nondeletional mechanism of thymic self tolerance. Science 246:1038.
21. Blackman, M.A., T.H. Finkel, J. Kappler, J. Cambier, and P. Marrack. 1991. Altered antigen receptor signalling in anergic T-cells from self-tolerant T-cell receptor B-chain transgenic mice. Proc. Natl. Acad. Sci. USA 88:6682.
22. LaSalle, J.M., K. Ota, and D.A. Hafler. 1991. Presentation of autoantigen by human T-cells. J. Immunol. 147:774.
23. Grosveld, F.G., T. Lund, E.J. Murray, A.L. Mellor, H.H.M. Dahl, and R.A. Flavell. The construction of cosmid libraries which can be used to transform eukaryotic cells. Nucl. Acids Res. 10:6715.
24. Brod, S.A., D. Benjamin, and D.A. Hafler. 1991. Restricted T-cell expression of IL2/IFN-γ mRNA in human inflammatory disease. J. Immunol. 147:810.
25. Freeman, G.J., G.S. Gray, C.D. Gimmi, D.B. Lombard, L-J. Zhou, M. White, J.D. Fingeroth, J.G. Gribben, and L.M. Nadler. 1991. Structure, expression, and T-cell costimulatory activity of the murine homologue of the human B lymphocyte activation antigen B7. J. Exp. Med. 174:625.
26. Altman, A.K.M. Coggeshall, and T. Mustelin. 1990. Molecular events mediating T-cell activation. Adv. Immunol. 48:227.
27. Bierer, B.E., P.S. Mattila, R.F. Standaert, L.A. Herzenberg, S.J. Burakoff, G. Crabtree, and S.L. Schreiber. 1990. Two distinct signal transmission pathways are inhibited by complexes formed between an immunophilin and either FK506 or rapamycin. Proc. Natl. Acad. Sci. USA. 87:9231.
28. Unanue, E.R., and P.M. Allen. 1987. The basis for the immunoregualtory role of macrophages and other accessory cells. Science 236:551.
29. Hewitt, C.R.A., and M. Feldman. 1989. Human T-cell clones present antigen. J. Immunol. 142:1429.
30. Lanzavecchia, A., E. Roosnek, T. Gregory, P. Berman, and S. Abrignani. 1988. T-cells can present antigens such as HIV gpl2O targeted to their own surface molecules. Nature, 334:530.
31. Gerrard, T.L., D.J. Volkman, C.H. Jurgensen, and A.S. Fauci 1986. Activated human T-cells can present denatured antigen. Hum. Immunol. 17:416.
32. Mueller, D.L., M.K. Jenkins, and R.H. Schwartz. 1989. An accessory cell-derived costimulatory signal acts independently of protein kinase C activation to allow T-cell proliferation and prevent the induction of unresponsiveness. J. Immunol., 142:2617.
33. Mueller, D.M., M.K. Jenkins, L. Chiodetti, and R.H. Schwartz. 1990. An intracellular calcium increase and protein kinase C activation fail to initiate T-cell proliferation in the absence of a costimulatory signal. J. Immunol. 144:3701.
34. O'Hehir, R.E., H. Yssel, S. Verma, J.E. de Vries, H. Spits, and J.R. Lamb. 1991. Clonal analysis of different lymphokine production in peptide and superantigen induced T-cell anergy. Internat. Immunol. 3:819.
35. O'Hehir, R.E., and J.R. Lamb. 1990. Induction of specific clonal anergy in human T lymphocytes by **Staphylococcus aureus enterotoxins**. Proc. Natl. Acad. Sci. USA. 87:8884.
36. Street, N., J.H. Schumacher, T.A.T. Fong, H. Bass., D.F. Fiorentino, J.A. Leverach, and T.R. Mossman 1990. Heterogeneity of mouse helper T-cells: evidence from bulk cultures and limiting dilution cloning for precursors Th1 and Th2 cells. J. Immunol. 144:1629.

The present invention has been illustrated by reference to specific Examples.

## Claims

1. A peptide consisting of all or a portion of fragment 84-102 of hMBP, said peptide comprising at least fragment 85-99 of hMBP, containing an alanine residue at position 85, 86, 87, 88, 89, 90, 91, 92, 93, 94, 95, 96, 97, 98 or 99, said peptide having the property of stimulating in vitro proliferation of T-cells that are derived from MS patients and that recognise the entire fragment 84-102 of hMBP.

2. The peptide of claim 1 wherein said portion is residues 85-99 of hMBP.

3. The peptide of claim 2 having an alanine substitution at position 90 (His), 91 (Phe) or 93 (Lys).

4. The peptide of claim 2 having an alanine substitution for the lysine present in MBP at position 93.

5. A pharmaceutical composition comprising an orally effective amount of a peptide according to any of claims 1, 2, 3 or 4 and a physiological acceptable carrier or diluent.

6. The composition of claim 5, wherein said effective amount is within the range of about 10 mg to 20 mg.

7. A pharmaceutical composition comprising a parenterally effective amount of a peptide according to any one of claims 1 to 4 and a physiologically acceptable carrier or diluent.

8. The composition of claim 7, wherein said effective amount is within the range of about 1 mg to 200 mg.

9. Use of a peptide according to any of claims 1 to 4 for preparing a medicament for altering immune function of CD4T-cells reactive with myelin basic protein in a mammal afflicted with multiple sclerosis wherein the peptide is administered via the parental route and is administered in an amount effective to alter said immune function.

10. Use of a peptide according to any of claims 1 to 4 for preparing a medicament for suppressing autoimmune response in a mammal, said autoimmune response being of the type observed in multiple sclerosis wherein the peptide is administered orally.

## Patentansprüche

1. Peptid, das aus dem gesamten Fragment 84-102 von hMBP oder einem Teil davon besteht, wobei das Peptid mindestens Fragment 85-99 von hMBP umfasst, das einen Alaninrest an Position 85, 86, 87, 88, 89, 90, 91, 92, 93, 94, 95, 96, 97, 98 oder 99 enthält, wobei das Peptid die Eigenschaft hat, in vitro die Proliferation von T-Zellen zu stimulieren, die von MS-Patienten stammen und die das gesamte Fragment 84-102 von hMBP erkennen.

2. Peptid nach Anspruch 1, wobei der Teil aus den Resten 85-99 von hMBP besteht.

3. Peptid nach Anspruch 2, das eine Alanin-Substitution an Position 90 (His), 91 (Phe) oder 93 (Lys) aufweist.

4. Peptid nach Anspruch 2, das eine Alanin-Substitution für das in MBP an Position 93 vorliegende Lysin aufweist.

5. Pharmazeutische Zusammensetzung, die eine oral wirksame Menge eines Peptids nach einem der Ansprüche 1, 2, 3 oder 4 und einen physiologisch verträglichen Träger oder ein physiologisch verträgliches Verdünnungsmittel umfasst.

6. Zusammensetzung nach Anspruch 5, wobei die wirksame Menge im Bereich von etwa 10 mg bis 20 mg liegt.

7. Pharmazeutische Zusammensetzung, die eine parenteral wirksame Menge eines Peptids nach einem der Ansprüche 1 bis 4 und einen physiologisch verträglichen Träger oder ein physiologisch verträgliches Verdünnungsmittel umfasst.

8. Zusammensetzung nach Anspruch 7, wobei die wirksame Menge im Bereich von etwa 1 mg bis 200 mg liegt.

9. Verwendung eines Peptids nach einem der Ansprüche 1 bis 4 zum Herstellen eines Medikaments zum Verändern der Immunfunktion von CD4T-Zellen, die mit myelin-basischem Protein reaktiv sind, bei einem Säuger, der an multipler Sklerose leidet, wobei das Peptid über die parenterale Route appliziert und in einer Menge verabreicht wird, die bewirkt, dass die Immunfunktion verändert wird.

10. Verwendung eines Peptids nach einem der Ansprüche 1 bis 4 zum Herstellen eines Medikaments zum Unterdrücken einer Autoimmunantwort bei einem Säuger, wobei es sich um die Art von Autoimmunantwort handelt, die bei multipler Sklerose festgestellt wird, wobei das Peptid oral verabreicht wird.

## Revendications

1. Peptide composé de la totalité ou d'une partie du fragment 84-102 de la PBMh, ledit peptide comprenant au moins le fragment 85-99 de la PBMh, contenant un résidu alanine en position 85, 86, 87, 88, 89, 90, 91, 92, 93, 94, 95, 96, 97, 98 ou 99, ledit peptide ayant la propriété de stimuler la prolifération in vitro de cellules T qui proviennent de patients atteints de sclérose en plaques et qui reconnaissent la totalité du fragment 84-102 de la PBMh.

2. Peptide selon la revendication 1, dans lequel ladite partie correspond aux résidus 85-99 de la PBMh.

3. Peptide selon la revendication 2, possédant une substitution alanine en position 90 (His), 91 (Phe) ou 93 (Lys).

4. Peptide selon la revendication 2, possédant une substitution alanine à la place de la lysine présente en position 93 dans la PBM.

5. Composition pharmaceutique comprenant une quantité efficace par voie orale d'un peptide selon l'une quelconque des revendications 1, 2, 3 ou 4 et un véhicule ou diluant physiologiquement acceptable.

6. Composition selon la revendication 5, dans laquelle ladite quantité efficace se situe dans la plage d'environ 10 mg à 20 mg.

7. Composition pharmaceutique comprenant une quantité efficace par voie parentérale d'un peptide selon l'une quelconque des revendications 1 à 4 ainsi qu'un véhicule ou diluant physiologiquement acceptable.

8. Composition selon la revendication 7, dans laquelle ladite quantité efficace se situe dans la plage d'environ 1 mg à 200 mg.

9. Utilisation d'un peptide selon l'une quelconque des revendications 1 à 4 pour préparer un médicament destiné à modifier la fonction immunitaire des cellules T-CD4 réactives vis-à-vis de la protéine basique de la myéline chez un mammifère atteint de sclérose en plaques, dans laquelle le peptide est administré par la voie parentérale et administré en une quantité efficace pour modifier ladite fonction immunitaire.

10. Utilisation d'un peptide selon l'une quelconque des revendications 1 à 4 pour préparer un médicament destiné à supprimer la réponse auto-immunitaire chez un mammifère, ladite réponse auto-immunitaire étant du type observé dans la sclérose en plaques, dans laquelle le peptide est administré par voie orale.
